# EUROPEAN PATENT SPECIFICATION

(11) **EP 4 577 120 B1**
(45) Date of publication and mention of the grant of the patent: **09.12.2026**
(21) Application number: 23758328.1
(22) Date of filing: 22.08.2023
(51) Int. Cl.: A61B 8/08, A61B 8/00

(54) **GUIDED ULTRASOUND IMAGING FOR POINT-OF-CARE STAGING OF MEDICAL CONDITIONS**
GEFÜHRTE ULTRASCHALLBILDGEBUNG ZUR EINSTUFUNG VON MEDIZINISCHEN ERKRANKUNGEN AM VERSORGUNGSORT
IMAGERIE ULTRASONORE GUIDÉE POUR STADIFICATION DE POINT D'INTERVENTION D'ÉTATS MÉDICAUX

(30) Priority: 25.08.2022 WO PCT/CN2022/114814; 24.10.2022 EP 22203355
(43) Date of publication of application: 02.07.2025
(73) Proprietor: Koninklijke Philips N.V., 5656 AE Eindhoven (NL)
(72) Inventor: XU, Jingping, 5656 AG Eindhoven (NL); DENG, Junping, 5656 AG Eindhoven (NL); SHIH, Cho-chiang, 5656 AG Eindhoven (NL)
(74) Representative: Philips Intellectual Property & Standards
(86) International application number: PCT/EP2023/072966
(87) International publication number: WO 2024/042044

(56) References cited:
- EP-A1- 3 936 054
- WO-A1-2020/020809
- US-A1- 2012 259 225
- US-A1- 2015 148 671
- US-A1- 2020 367 853
- US-A1- 2022 237 798

## Description

### FIELD OF THE INVENTION

The present disclosure relates generally to ultrasound imaging. In particular, an ultrasound system provides guidance to acquire ultrasound images of multiple views, automatically extracts features from the ultrasound images, assigns sub-scores to the features, and determines a staging value of a medical condition of the patient.

### BACKGROUND OF THE INVENTION

Physicians use many different medical diagnostic systems and tools to monitor a subject's health and diagnose and treat medical conditions. Ultrasound imaging systems are widely used for medical imaging and measurement. The ultrasound transducer probe may include an array of ultrasound transducer elements that transmit acoustic waves into a subject's body and record acoustic waves reflected and backscattered from the internal anatomical structures within the subject's body, which may include tissues, blood vessels, and internal organs. The transmission and reception of acoustic waves, along with various beamforming and processing techniques, create an image of the subject's internal anatomical structures.

Ultrasound imaging is a safe, useful, and in some applications, non-invasive tool for diagnostic examination, interventions, and/or treatment. Ultrasound imaging can be used to diagnose non-alcoholic fatty liver disease (NAFLD), including a stage of progression of NAFLD. Accurately diagnosing an NAFLD stage typically requires advanced quantitative ultrasound (QUS) technology not available in point-of-care (POC) ultrasound systems due to computational or hardware limitations. In addition, acquiring NAFLD images of sufficient quality and analyzing such images typically requires a high level of expertise by the user as well as optimal conditions for the procedure, including sufficient time and resources. A lack of expertise, time, or resources can easily lead to poor quality ultrasound images and inaccurate measurements and diagnoses as a result.

EP 3936054 A1 discloses a method for determinign a prognosis of the risk of bronchopulmonary dysplasia in premature infants based on adjusting a score obtained from lung ultrasound with the gestational age of the infant.

WO 2020/020809 A1 discloses an ultrasound imaging system and method for ultrasonically inspecting biological tissue, such as liver, and for automatically identifying and acquiring a view suitable for hepatic-renal echo-intensity ratio quantification using one or more neural networks.

### SUMMARY OF THE INVENTION

Aspects of the present disclosure are systems, devices, and methods for point-of-care staging of medical conditions through guided ultrasound imaging. Aspects of the present disclosure advantageously guide a non-expert user to obtain ultrasound images of a patient anatomy including multiple views of sufficient quality for analysis. Additionally, a staging value related to the progression of the medical condition is automatically determined based on the acquired ultrasound images. Aspects of the present disclosure also advantageously display to the user image features within the acquired ultrasound images and corresponding sub-scores associated with each image feature. By displaying to the user the images acquired and/or selected, as well as the corresponding sub-scores, the user can be more confident that the resulting staging value is accurate. Aspects of the present disclosure also advantageously reduces time and effort for the physician or user of the system in reporting by automatically generating a report.

The invention is defined by the independent claims. The dependent claims define advantageous embodiments.

In some aspects of the disclosure, an ultrasound imaging system may provide a non-expert user in a point-of-care setting instructions on how to obtain ultrasound images of the patient anatomy from multiple views. The system may analyze the images to ensure that they are of sufficient quality and select at least one image for each view. The ultrasound image may then extract image features corresponding to each view and assign a feature sub-score to each selected image. The ultrasound system may then display the selected images and associated sub-scores as well as determine the staging value for the medical condition based on the images and sub-scores.

According to an exemplary aspect, an ultrasound system is provided. The system includes a processor configured for communication with a display and a transducer array of an ultrasound probe, wherein the processor is configured to: control the transducer array to obtain a first ultrasound image corresponding to a first view of a patient anatomy and a second ultrasound image of a patient anatomy corresponding to a second view of the patient anatomy; identify a first image feature associated with a medical condition of the patient anatomy within the first ultrasound image and a second image feature associated with the medical condition within the second ultrasound image; determine a first sub-score for the first image feature and a second sub-score for the second image feature; determine a staging value representative of a progression of the medical condition based on the first sub-score and the second sub-score; and output, to the display, a screen display comprising: the staging value; and at least one of: the first ultrasound image, an indication of the first image feature in the first ultrasound image, and the first sub-score; or the second ultrasound image, an indication of the second image feature in the second ultrasound image, and the second sub-score.

In some aspects, the processor is configured to output, to the display, user guidance to obtain the first ultrasound image corresponding to the first view of the patient anatomy. In some aspects, the user guidance comprises a graphical representation of a probe and/or orientation for the ultrasound probe. In some aspects, the user guidance comprises a reference image associated with the first view of the patient anatomy. In some aspects, the user guidance comprises a description of a dynamic behavior associated with the first view of the patient anatomy. In some aspects, the processor is configured to determine a quality associated with the first ultrasound image before identifying the first image feature within the first ultrasound image. In some aspects, the processor is configured to determine the quality based on a comparison between the first ultrasound image and a reference image associated with the first view of the patient anatomy. In some aspects, the processor is configured to: if the quality satisfies a threshold, identify the first image feature within the first ultrasound image; if the quality does not satisfy the threshold, control the transducer array to obtain a further ultrasound image corresponding to the first view of the patient anatomy. In some aspects, to determine the first sub-score and the second sub-score, the processor is configured to implement a first machine learning algorithm. In some aspects, the first machine learning algorithm comprises a multi-task learning model. In some aspects, to identify the first image feature and the second image feature, the processor is configured to implement a second machine learning algorithm different than the first machine learning algorithm. In some aspects, the patient anatomy comprises a liver, and the medical condition comprises hepatic steatosis. In some aspects, the first sub-score for the first image feature and the second sub-score for the second image feature correspond to ultrasonographic fatty liver indicator (US-FLI). In some aspects, the first image feature and the second feature each comprise a different one of: liver-kidney contrast, posterior attenuation, vessel blurring, gallbladder visualization, diaphragmatic attenuation visualization, or focal sparing.

According to an exemplary aspect, a computer-implemented method is provided. The method includes controlling a transducer array of an ultrasound imaging probe to obtain a first ultrasound image corresponding to a first view of a patient anatomy and a second ultrasound image of a patient anatomy corresponding to a second view of the patient anatomy; identifying a first image feature associated with a medical condition of the patient anatomy within the first ultrasound image and a second image feature associated with the medical condition within the second ultrasound image; determining a first sub-score for the first image feature and a second sub-score for the second image feature; determining a staging value representative of a progression of the medical condition based on the first sub-score and the second sub-score; and outputting, to the display, a screen display comprising: the staging value; and at least one of: the first ultrasound image, an indication of the first image feature in the first ultrasound image, and the first sub-score; or the second ultrasound image, an indication of the second image feature in the second ultrasound image, and the second sub-score.

Additional aspects, features, and advantages of the present disclosure will become apparent from the following detailed description.

### BRIEF DESCRIPTION OF THE DRAWINGS

Illustrative aspects of the present disclosure will be described with reference to the accompanying drawings, of which:
Fig. 1 is a schematic diagram of an ultrasound imaging system, according to aspects of the present disclosure.
Fig. 2 is a schematic diagram of a processor circuit, according to aspects of the present disclosure.
Fig. 3 is a flow diagram of a method of obtaining and analyzing ultrasound images corresponding to multiple views of a patient anatomy, according to aspects of the present disclosure.
Fig. 4 is a diagrammatic view of a graphical user interface displaying user guidance for obtaining ultrasound images for diagnosis, according to aspects of the present disclosure.
Fig. 5 is a diagrammatic view of a table of user guidance for obtaining ultrasound images of different views for diagnosis, according to aspects of the present disclosure.
Fig. 6 is a diagrammatic view of acquired ultrasound images of multiple views and corresponding reference images for comparison, according to aspects of the present disclosure.
Fig. 7 is a diagrammatic view of acquired ultrasound images and identified features, according to aspects of the present disclosure.
Fig. 8 is a schematic diagram of a machine learning algorithm, according to aspects of the present disclosure.
Fig. 9A is a diagrammatic view of a graphical user interface displaying a selected ultrasound image for a particular view, a feature, and a score, according to aspects of the present disclosure.
Fig. 9B is a diagrammatic view of a graphical user interface displaying a selected ultrasound image for a particular view, a feature, and a score, according to aspects of the present disclosure.
Fig. 9C is a diagrammatic view of a graphical user interface displaying a selected ultrasound image for a particular view, a feature, and a score, according to aspects of the present disclosure.
Fig. 9D is a diagrammatic view of a graphical user interface displaying a selected ultrasound image for a particular view, a feature, and a score, according to aspects of the present disclosure.
Fig. 9E is a diagrammatic view of a graphical user interface displaying a selected ultrasound image for a particular view, a feature, and a score, according to aspects of the present disclosure.
Fig. 9F is a diagrammatic view of a graphical user interface displaying a selected ultrasound image for a particular view, a feature, and a score, according to aspects of the present disclosure.
Fig. 10 is a diagrammatic view of a graphical user interface displaying ultrasound images, corresponding scores, and a staging value, according to aspects of the present disclosure.
Fig. 11 is a diagrammatic view of a graphical user interface displaying an ultrasound image, corresponding scores, and a stage score, according to aspects of the present disclosure.
Fig. 12 is a flow diagram of a method of determining a staging value of a medical condition, according to aspects of the present disclosure.

### DETAILED DESCRIPTION OF EMBODIMENTS

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

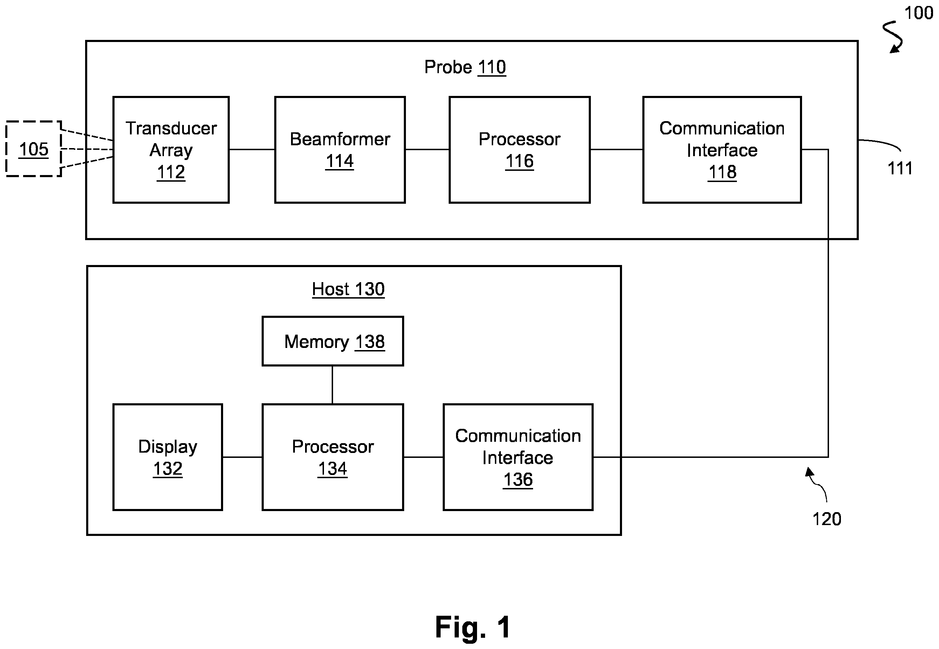

Fig. 1 is a schematic diagram of an ultrasound imaging system 100, according to aspects of the present disclosure. The system 100 is used for scanning an area or volume of a subject's body. A subject may include a patient of an ultrasound imaging procedure, or any other person, or any suitable living or non-living organism or structure. The system 100 includes an ultrasound imaging probe 110 in communication with a host 130 over a communication interface or link 120. The probe 110 may include a transducer array 112, a beamformer 114, a processor circuit 116, and a communication interface 118. The host 130 may include a display 132, a processor circuit 134, a communication interface 136, and a memory 138 storing subject information.

In some aspects, the probe 110 is an external ultrasound imaging device including a housing 111 configured for handheld operation by a user. The transducer array 112 can be configured to obtain ultrasound data while the user grasps the housing 111 of the probe 110 such that the transducer array 112 is positioned adjacent to or in contact with a subject's skin. The probe 110 is configured to obtain ultrasound data of anatomy within the subject's body while the probe 110 is positioned outside of the subject's body for general imaging, such as for abdomen imaging, liver imaging, etc. In some aspects, the probe 110 can be an external ultrasound probe, a transthoracic probe, and/or a curved array probe.

In other aspects, the probe 110 can be an internal ultrasound imaging device and may comprise a housing 111 configured to be positioned within a lumen of a subject's body for general imaging, such as for abdomen imaging, liver imaging, etc. In some aspects, the probe 110 may be a curved array probe. Probe 110 may be of any suitable form for any suitable ultrasound imaging application including both external and internal ultrasound imaging.

In some aspects, aspects of the present disclosure can be implemented with medical images of subjects obtained using any suitable medical imaging device and/or modality. Examples of medical images and medical imaging devices include x-ray images (angiographic images, fluoroscopic images, images with or without contrast) obtained by an x-ray imaging device, computed tomography (CT) images obtained by a CT imaging device, positron emission tomography-computed tomography (PET-CT) images obtained by a PET-CT imaging device, magnetic resonance images (MRI) obtained by an MRI device, single-photon emission computed tomography (SPECT) images obtained by a SPECT imaging device, optical coherence tomography (OCT) images obtained by an OCT imaging device, and intravascular photoacoustic (IVPA) images obtained by an IVPA imaging device. The medical imaging device can obtain the medical images while positioned outside the subject body, spaced from the subject body, adjacent to the subject body, in contact with the subject body, and/or inside the subject body.

For an ultrasound imaging device, the transducer array 112 emits ultrasound signals towards an anatomical object 105 of a subject and receives echo signals reflected from the object 105 back to the transducer array 112. The ultrasound transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a subject's anatomy. In some aspects, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy or anatomical feature, such as a kidney, liver, and/or any other anatomy of a subject. The present disclosure can be implemented in the context of any number of anatomical locations and tissue types, including without limitation, organs including the liver, kidneys, gall bladder, pancreas, lungs; ducts; intestines; nervous system structures including the brain, dural sac, spinal cord and peripheral nerves; the urinary tract; as well as valves within the blood vessels, blood, abdominal organs, and/or other systems of the body. In some aspects, the object 105 may include malignancies such as tumors, cysts, lesions, hemorrhages, or blood pools within any part of human anatomy. The anatomy may be a blood vessel, as an artery or a vein of a subject's vascular system, including cardiac vasculature, peripheral vasculature, neural vasculature, renal vasculature, and/or any other suitable lumen inside the body. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. In some aspects, the beamformer 114 may apply a time-delay to signals sent to individual acoustic transducers within an array in the transducer 112 such that an acoustic signal is steered in any suitable direction propagating away from the probe 110. The beamformer 114 may further provide image signals to the processor circuit 116 based on the response of the received ultrasound echo signals. The beamformer 114 may include multiple stages of beamforming. The beamforming can reduce the number of signal lines for coupling to the processor circuit 116. In some aspects, the transducer array 112 in combination with the beamformer 114 may be referred to as an ultrasound imaging component.

The processor 116 is coupled to the beamformer 114. The processor 116 may also be described as a processor circuit, which can include other components in communication with the processor 116, such as a memory, beamformer 114, communication interface 118, and/or other suitable components. The processor 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 116 may also be implemented as a combination of computing devices, e.g., a combination of a DSP and a microprocessor, a plurality of microprocessors, one or more microprocessors in conjunction with a DSP core, or any other such configuration. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 WiFi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor 134 is coupled to the communication interface 136. The processor 134 may also be described as a processor circuit, which can include other components in communication with the processor 134, such as the memory 138, the communication interface 136, and/or other suitable components. The processor 134 may be implemented as a combination of software components and hardware components. The processor 134 may include a central processing unit (CPU), a graphics processing unit (GPU), a digital signal processor (DSP), an application-specific integrated circuit (ASIC), a controller, an FPGA device, another hardware device, a firmware device, or any combination thereof configured to perform the operations described herein. The processor 134 may also be implemented as a combination of computing devices, e.g., a combination of a DSP and a microprocessor, a plurality of microprocessors, one or more microprocessors in conjunction with a DSP core, or any other such configuration. The processor 134 can be configured to generate image data from the image signals received from the probe 110. The processor 134 can apply advanced signal processing and/or image processing techniques to the image signals. In some aspects, the processor 134 can form a three-dimensional (3D) volume image from the image data. In some aspects, the processor 134 can perform real-time processing on the image data to provide a streaming video of ultrasound images of the object 105. In some aspects, the host 130 includes a beamformer. For example, the processor 134 can be part of and/or otherwise in communication with such a beamformer. The beamformer in the in the host 130 can be a system beamformer or a main beamformer (providing one or more subsequent stages of beamforming), while the beamformer 114 is a probe beamformer or micro-beamformer (providing one or more initial stages of beamforming).

The memory 138 is coupled to the processor 134. The memory 138 may be any suitable storage device, such as a cache memory (e.g., a cache memory of the processor 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory.

The memory 138 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 138 may be located within the host 130. Subject information may include measurements, data, files, other forms of medical history, such as but not limited to ultrasound images, ultrasound videos, and/or any imaging information relating to the subject's anatomy. The subject information may include parameters related to an imaging procedure such as an anatomical scan window, a probe orientation, and/or the subject position during an imaging procedure. The memory 138 can also be configured to store information related to the training and implementation of machine learning algorithms (e.g., neural networks) and/or information related to implementing image recognition algorithms for detecting/segmenting anatomy, image quantification algorithms, and/or image acquisition guidance algorithms, including those described herein.

The display 132 is coupled to the processor circuit 134. The display 132 may be a monitor or any suitable display. The display 132 is configured to display the ultrasound images, image videos, and/or any imaging information of the object 105.

The system 100 may be used to assist a sonographer in performing an ultrasound scan. The scan may be performed in a at a point-of-care setting. In some instances, the host 130 is a console or movable cart. In some instances, the host 130 may be a mobile device, such as a tablet, a mobile phone, or portable computer. During an imaging procedure, the ultrasound system can acquire an ultrasound image of a particular region of interest within a subject's anatomy. The ultrasound system 100 may then analyze the ultrasound image to identify various parameters associated with the acquisition of the image such as the scan window, the probe orientation, the subject position, and/or other parameters. The system 100 may then store the image and these associated parameters in the memory 138. At a subsequent imaging procedure, the system 100 may retrieve the previously acquired ultrasound image and associated parameters for display to a user which may be used to guide the user of the system 100 to use the same or similar parameters in the subsequent imaging procedure, as will be described in more detail hereafter.

In some aspects, the processor 134 may utilize deep learning-based prediction networks to identify parameters of an ultrasound image, including an anatomical scan window, probe orientation, subject position, and/or other parameters. In some aspects, the processor 134 may receive metrics or perform various calculations relating to the region of interest imaged or the subject's physiological state during an imaging procedure. These metrics and/or calculations may also be displayed to the sonographer or other user via the display 132.

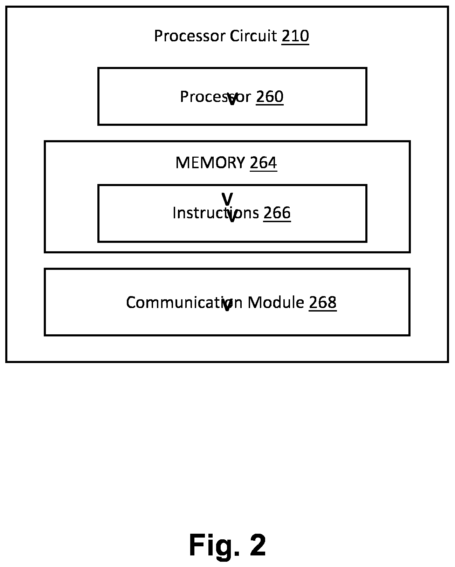

Fig. 2 is a schematic diagram of a processor circuit, according to aspects of the present disclosure. One or more processor circuits can be configured to carry out the operations described herein. The processor circuit 210 may be implemented in the probe 110, the host system 130 of Fig. 1, or any other suitable location. For example, the processor 116 of the probe 110 can be part of the processor circuit 210. For example, the processor 134 and/or the memory 138 can be part of the processor circuit 210. In an example, the processor circuit 210 may be in communication with the transducer array 112, beamformer 114, communication interface 118, communication interface 136, and/or the display 132, as well as any other suitable component or circuit within ultrasound system 100. As shown, the processor circuit 210 may include a processor 260, a memory 264, and a communication module 268. These elements may be in direct or indirect communication with each other, for example via one or more buses.

The processor 260 may include a CPU, a GPU, a DSP, an application-specific integrated circuit (ASIC), a controller, an FPGA, another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration. The processor 260 may also include an analysis module as will be discussed in more detail hereafter. The analysis module may implement various machine learning algorithms and may be a hardware or a software implementation. The processor 260 may additionally include a preprocessor in either hardware or software implementation. The processor 260 may execute various instructions, including instructions stored on a non-transitory computer readable medium, such as the memory 264.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In some instances, the memory 264 includes a non-transitory computer-readable medium. The memory 264 may store instructions 266. The instructions 266 may include instructions that, when executed by the processor 260, cause the processor 260 to perform the operations described herein with reference to the probe 110 and/or the host 130 (Fig. 1). Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 266 may include various aspects of a preprocessor, machine learning algorithm, convolutional neural network (CNN) or various other instructions or code. In some aspect, the memory 264 may be or include a non-transitory computer readable medium.

The communication module 268 can include any electronic circuitry and/or logic circuitry to facilitate direct or indirect communication of data between the processor circuit 210, the probe 110, and/or the host 130. In that regard, the communication module 268 can be an input/output (I/O) device. In some instances, the communication module 268 facilitates direct or indirect communication between various elements of the processor circuit 210 and/or the probe 110 (Fig. 1) and/or the host 130 (Fig. 1).

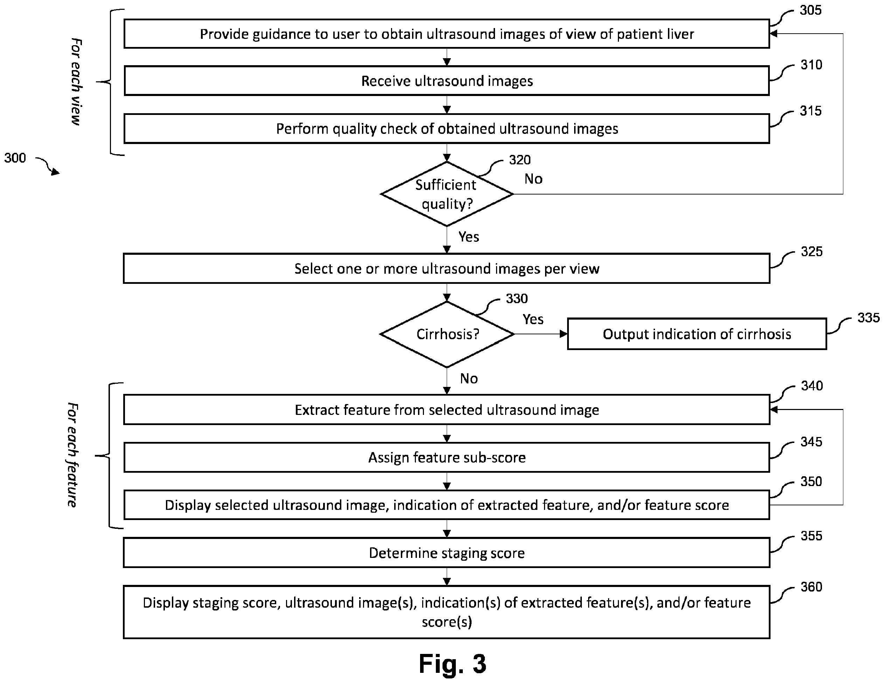

Fig. 3 is a flow diagram of a method 300 of obtaining and analyzing ultrasound images corresponding to multiple views of a patient anatomy, according to aspects of the present disclosure. As illustrated, the method 300 includes a number of enumerated steps, but aspects of the method 300 may include additional steps before, after, or in between the enumerated steps. In some aspects, one or more of the enumerated steps may be omitted, performed in a different order, or performed concurrently. The steps of the method 300 can be carried out by any suitable component within the system 100 and all steps need not be carried out by the same component. In some aspects, one or more steps of the method 300 can be performed by, or at the direction of, a processor circuit, including, e.g., the processor 116 (Fig. 1), the processor 134 (Fig. 1), the processor 260 (Fig. 2) or any other suitable component.

Aspects of the method 300 may describe methods of providing guidance to non-expert ultrasound imaging system users to acquire high quality ultrasound images. As will be explained hereafter, these ultrasound images may include multiple views of a region of a patient's anatomy. In addition, aspects of the present disclosure relate to extracting features within the acquired ultrasound images. This extraction may include indicating regions of interest within each ultrasound image. The features extracted may be referred to as image features and may be signs, indicators, or symptoms of how a medical condition manifests itself in ultrasound images. These image features may reflect the severity and/or progression of a particular medical condition. In some aspects, hepatic steatosis (HS) may be a medical condition identified by the ultrasound imaging system. HS may include alcoholic fatty liver disease, as well as non-alcoholic fatty liver disease (NAFLD). In some aspects, NAFLD may correspond to obesity and lead to various liver diseases. In this way, diagnosing NAFLD, other forms of HS, or any other condition, may be accomplished by a non-expert ultrasound user in a point-of-care setting thus expanding the availability of technology capable of diagnosing these conditions. By way of example, a stage of progression may be determined for other medical conditions including diffuse-type disease, such as in the liver and/or other organs, and/or other diseases in other organs.

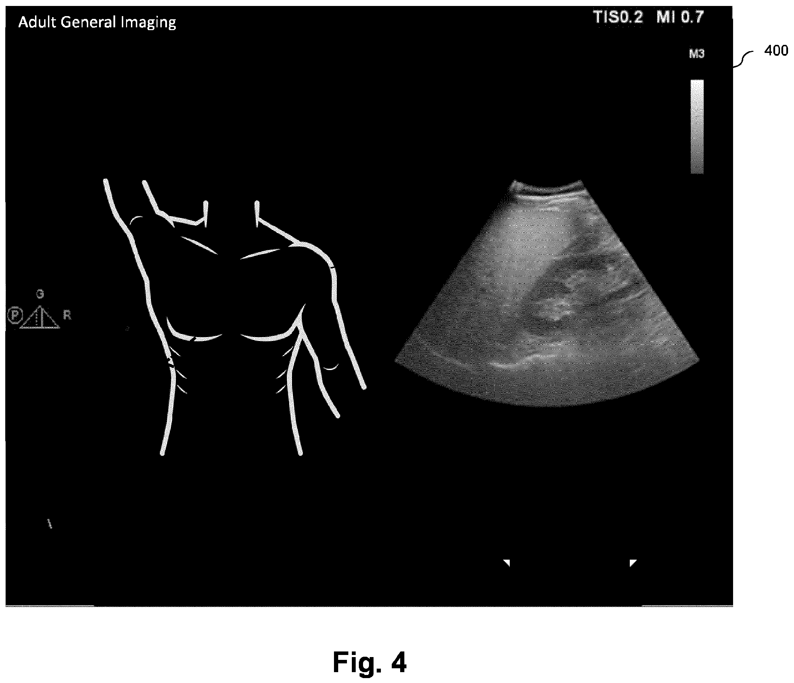

At step 305, the method 300 includes providing guidance to a user to obtain ultrasound images of a view of a patient liver. Guidance to the user to obtain ultrasound images of a view of the patient liver may be of any form. For example, a processor circuit of the ultrasound system may be in communication with a display. The processor circuit (e.g., the processor circuit 210) may output to the display a graphical user interface including user guidance. In some aspects, as will be described with reference to Fig. 4, the user guidance may include pictorial graphics of an exemplary patient, as well as an ultrasound system probe and field of view. In some aspects, the user guidance may include text describing the position of the ultrasound probe in relation to the patient's body. In some aspects, the graphical user interface displayed to the non-expert user may include graphics and/or text which may inform the non-expert user where to place the probe, how to move the probe, including in which direction to move the probe as well as various rotational positions, or any other way.

In some aspects, guidance displayed to the user may include highlighting or outlining the features within acquired images in real time. For example, a live imaging loop may be displayed to the non-expert user as the user positions the ultrasound imaging probe adjacent to the patient's body. As live images are displayed, outlines of various features within the live images may be displayed to assist the user in positioning the ultrasound imaging probe in the correct location.

In some aspects, user guidance may include the display of a reference image. For example, the user of the ultrasound system may acquire ultrasound images in real time and compare the acquired ultrasound images to the reference image to determine whether the probe is positioned in the correct position relative to the patient's body.

In some aspects, user guidance may additionally include an indication of the expected dynamic behavior of the patient anatomy during live imaging. For example, movement of particular structures including the liver, kidney, diaphragm, etc., or any other features within the patient anatomy may be identified and described. This dynamic behavior may be described with text, as well as with exemplary images including live images or a series of images including a video clip.

Additional aspects of providing guidance to a non-expert user to acquire high quality ultrasound images of multiple views of the patient anatomy will be described in more detail with reference to Figs. 4-5 hereafter.

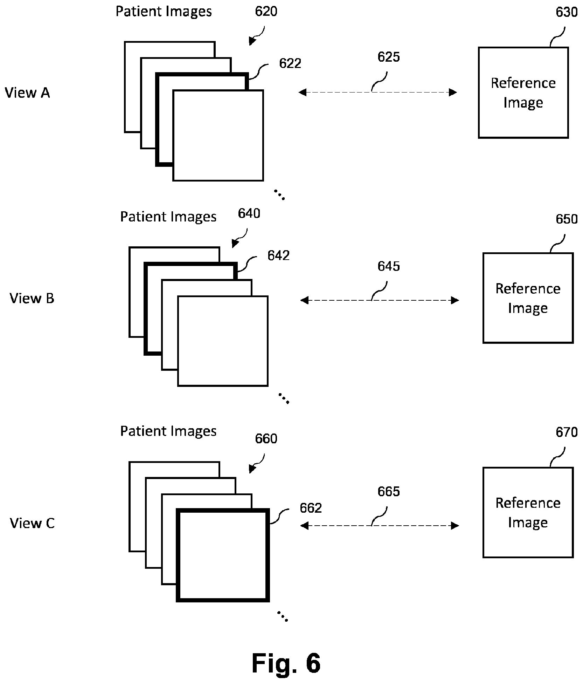

At step 310, the method 300 includes receiving ultrasound images. The ultrasound images received at the step 310 may include any suitable ultrasound images. For example, in some aspects, the ultrasound images received may include B-mode images. Additionally, any suitable type of ultrasound imaging system may be used to acquire the ultrasound images at step 310. For example an ultrasound imaging system may be similar to the ultrasound imaging system 100 described with reference to Fig. 1. At step 315, the method 300 includes performing a quality check of the ultrasound images. At step 315, the ultrasound imaging system 100 may ensure that the images received at the step 310 are of sufficient quality to perform the subsequent steps of the method 300. Performing a quality check of the received ultrasound images may include comparing the ultrasound images to corresponding reference images, as well as determining whether features of interest are present within ultrasound images, including whether the entire feature is present or a portion of the feature is present. In some aspects, the quality check of the received ultrasound images may be performed by a user of the ultrasound imaging system, or a machine learning network of the ultrasound imaging system 100. For example, a machine learning network may be trained to analyze and/or compare received ultrasound images and assign a score relating to the quality of the ultrasound images. Additional aspects of performing quality checks of obtained ultrasound images will be described with reference to Figs. 6-8 hereafter.

It is noted that the method 300 may be configured to identify multiple features within multiple ultrasound images received. For example, if the method 300 is implemented to determine a staging score for NAFLD, the method 300 may assist the user in obtaining multiple images corresponding to multiple views of the liver kidney region of the patient anatomy. In some aspects, six features of the liver-kidney region of the patient anatomy may indicate the degree of NAFLD within a patient. The method 300 may be configured to extract these six features within the obtained ultrasound images. As will be explained hereafter, it is contemplated that any number of features may be extracted corresponding to any suitable medical condition. In the example of identifying six features associated with NAFLD, the steps 305, 310, and 315 may be performed multiple times for each of the six features. For example, at the first time the step 305 is performed, the processor circuit 210 of the ultrasound imaging system 100 may provide guidance to the system user to obtain ultrasound images related to a feature of liver-kidney contrast. At the step 310, these ultrasound images may be obtained, and at the step 315, these ultrasound images may be analyzed to ensure that they are of sufficient quality. After the performance of step 315 in relation to the first feature liver-kidney contrast, the processor circuit 210 may revert to the step 305 and perform the steps 305, 310, and 315 in relation to a second feature, for example, posterior attenuation. The processor circuit 210 may then perform the steps 310 and 315 to receive additional ultrasound images relating to this feature of posterior attenuation, and then the processor circuit 210 may again revert to the step 305 relating to an additional feature, and so on. In some aspects, the processor circuit 210 may perform any subset of the steps shown and described with reference to the method 300 of Fig. 3 for each identified feature before repeating any of those same steps for an additional feature.

At step 320, the method 300 includes determining whether the ultrasound images are of sufficient quality. Determining whether the ultrasound images are sufficient quality may include any suitable process. For example, a guidance toolkit may be provided to assist the user in checking if the acquired images are of sufficient quality. In some aspects, image quality may be performed visually by comparing current acquired images with reference images. An inexperienced user may be provided with a reference image adjacent to acquired images for comparison. In some examples, an experienced user may verify the image quality of the obtained images without reference to the reference image. In some aspects, the ultrasound imaging system 100 may automatically perform an image quality check with various algorithms or modules. For example, the processor circuit 210 of the ultrasound imaging system 100 may be configured to implement a machine learning algorithm or image processing techniques to automatically assess the quality of a particular image.

At step 325, the method 300 includes selecting one or more ultrasound images per view. As described with reference to the step 315 previously, multiple features of the target region of the patient anatomy may be necessary to perform an accurate staging of a medical condition of the patient. In this way, different views of the patient anatomy may better illustrate different features. For example, a one-to-one relationship may exist between views and features. For purposes of the present disclosure, a view may correspond to a probe position relative to the patient anatomy. In this way, for each feature to be extracted from acquired ultrasound images, the ultrasound imaging system 100 may provide different guidance to the user to position the ultrasound imaging probe at different locations relative to the patient (e.g., at step 305).

However, at the step 310, multiple ultrasound images may be received corresponding to the same view or probe position. As a result, the ultrasound imaging system 100 may select one ultrasound image corresponding to one view for each of the views of the patient anatomy. In some aspects, the ultrasound imaging system may be configured to select additional ultrasound images for a single view.

Selecting one or more ultrasound images for each view may be accomplished by comparing each of the ultrasound images associated with a reference image. For example, the ultrasound imaging system user may examine each of the ultrasound images associated with a single view and compare them with the reference image. The ultrasound imaging system user may then select the ultrasound image of the multiple ultrasound images associated with the view that corresponds most closely to the reference image. In some aspects, the ultrasound imaging system 100 may automatically select the one or more ultrasound images for each view. For example, various image processing techniques, as well as machine learning algorithms, may be implemented to select an image of the multiple ultrasound images received related to a particular view which corresponds most closely to the reference image. Additional aspects of selecting one or more ultrasound images for each view will be described in more detail with reference to Fig. 6.

At step 330, the method 300 includes determining whether the selected ultrasound image shows cirrhosis. In some implementations, determining whether the selected ultrasound image shows cirrhosis includes analyzing the liver background within the selected image. **In** some aspects, elastography may be used to determine if the liver background indicates cirrhosis. **In** implementations in which elastography is unavailable, the liver background of the ultrasound image could be used to identify if the liver background corresponds to a normal or healthy state or if the liver background indicates cirrhosis.

**In** some aspects, the step 330 may be performed by analyzing any one of the ultrasound images selected at the step 325. For example, in an implementation in which six features are selected and six ultrasound images are selected at the step 325, the processor circuit 210 of the ultrasound imaging system 100 may analyze each of these six ultrasound images at the step 330 to determine whether the liver background indicates cirrhosis. **In** some aspects, only one of these six ultrasound images may be selected. In still other aspects, a separate ultrasound image may be selected and analyzed at the step 330, including any of the ultrasound images received at the step 310.

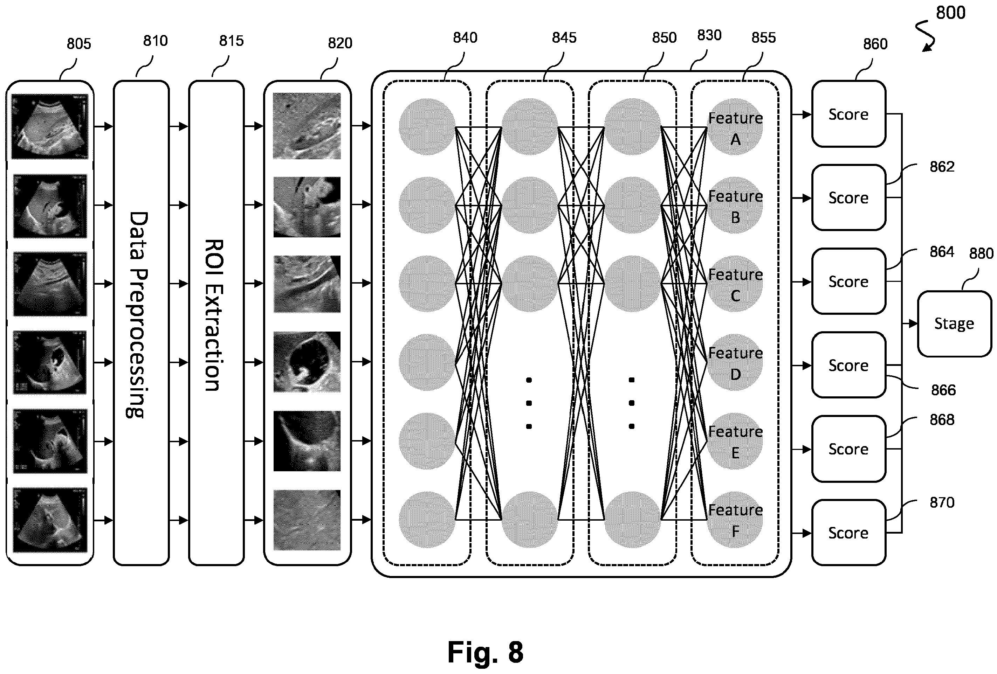

It is noted that any of the machine learning algorithms described herein may be configured to perform the step 330. For example, the ultrasound imaging system 100, as well as any machine learning algorithm, such as the algorithm 800 described with reference to Fig. 8, may include a cirrhosis analysis module. This cirrhosis analysis module may be configured to determine whether the ultrasound images received at the step 310 and or selected at the step 325 indicates cirrhosis.

At step 335, the method 300 includes outputting an indication of cirrhosis. In some examples, assigning a staging value of NAFLD, or any other medical condition, may not be sufficiently accurate if the ultrasound images received at the step 310 indicates cirrhosis. In such an example, the method 300 proceeds to the step 335. The step 335 includes indicating to the user that correct liver staging may not be provided in this situation with the ultrasound imaging system 100 and associated method. In this way, the method 300 may end at the step 335.

In some aspects, after the step 335 is completed, the ultrasound imaging system 100 may further generate a report for the user of the ultrasound imaging system, the patient, or another physician, indicating that the ultrasound images indicated cirrhosis and that the method 300 was not completed in its entirety.

At step 340, the method 300 includes extracting a feature from the selected ultrasound image. The method 300 may proceed to the step 340 if, at the step 330, it is determined that the ultrasound images obtained at the step 310 do not indicate cirrhosis. As described previously with reference to steps 305-315, the steps 340-350 may be performed multiple times for each identified feature. In an example in which the method 300 is implemented to diagnose a staging value of NAFLD and the processor circuit 210 is configured to identify and extract 6 features, the processor circuit 210 may identify the ultrasound image corresponding to the first feature (e.g., liver-kidney contrast) and extract this feature from the selected ultrasound image at the step 340. After then completing the steps 345 (described hereafter), the processor circuit 210 may identify the ultrasound image corresponding to the second feature (e.g., posterior attenuation) and perform the steps 340-350 again. This process may continue for each feature or view until all the selected ultrasound images corresponding to the different views have been analyzed according to the steps 340-350.

The processor circuit 210 may extract features from ultrasound images in any suitable way. For example, as will be explained in more detail hereafter with reference to Fig. 8, the processor circuit 210 may implement a machine learning algorithm to extract features from the selected ultrasound images. In some instances, the processor circuit 210 may apply various pre-processing techniques to a selected ultrasound image prior to extracting features. For example, the processor circuit 210 may adjust various characteristics of the selected image, such as, for example, an image contrast, brightness, image size, coloration, or any other characteristic. In some instances, the processor circuit 210 may additionally apply various image processing techniques to the selected ultrasound image prior to or after implementation of the machine learning network. For example, the processor circuit 210 may perform various image processing techniques, such as edge identification of features within the selected ultrasound images, pixel-by-pixel analysis to determine transition between light pixels and dark pixels, filtering, or any other suitable techniques to determine the location of various organs or structures shown within the selected ultrasound images.

At step 345, the method 300 includes assigning a feature score. In some aspects, the processor circuit 210 may assign a feature score to the selected ultrasound image by implementing a machine learning algorithm. The machine learning algorithm which assigns a score to the selected ultrasound image after the corresponding feature has been extracted may be the same machine learning algorithm which may be used to extract the features at the step 340. In some aspects, the machine learning algorithm for assigning a features score may be a separate algorithm.

The scoring convention implemented by the processor circuit 210 may be of any suitable type. For example, the processor circuit 210 may be configured to assign scores to features and/or ultrasound images using the ultrasonographic fatty liver indicator (US-FLI) scoring system. For example, the features which the processor circuit 210 is configured to extract may include liver-kidney contrast, posterior attenuation, portal vein blurring, gallbladder wall visualization, diaphragm definition, and focal fat sparing. As noted, any suitable number or types of features may be extracted from the ultrasound images selected, including any features related to different medical conditions. In this example, the liver-kidney contrast features may be assigned an integer score on the scale of 0-3, where a score of 0 corresponds to an isoechoic region, and a score of 3 corresponds to a severely hyperechoic region. The remaining features, including the posterior attenuation, portal vein blurring, gallbladder wall visualization, diaphragm definition, and focal fat sparing, may be assigned an integer score of 0 or 1. For example, posterior attenuation may be assigned a score of 1 if the ultrasound image shows attenuation, and 0 if the ultrasound image shows no attenuation. The portal vein blurring feature may be assigned a score of 1 if vessel blurring is present within the selected ultrasound image and 0 if vessel blurring is not present. The gallbladder wall visualization score may be a 1 if it is difficult to visualize the gallbladder wall and 0 if the gallbladder wall is easily visualized. The diaphragm definition feature may be assigned a score of 1 if the diaphragm is obscured in the corresponding ultrasound image and 0 if the diaphragm is clearly defined. The focal fat sparing feature may be assigned a score of 1 if focal fat sparing is present in the corresponding ultrasound image and 0 if focal fat sparing is not present.

For any of the scoring conventions described above relating to the six features of NAFLD, the score ranges could be different, including any suitable range. **In** addition, assigned scores could be non-integer numbers. Other scoring conventions may include scoring features with percentages, measurements or values of features within the selected ultrasound image, such as an estimated volume or weight of fat or other tissue or material, as well as any other scoring conventions.

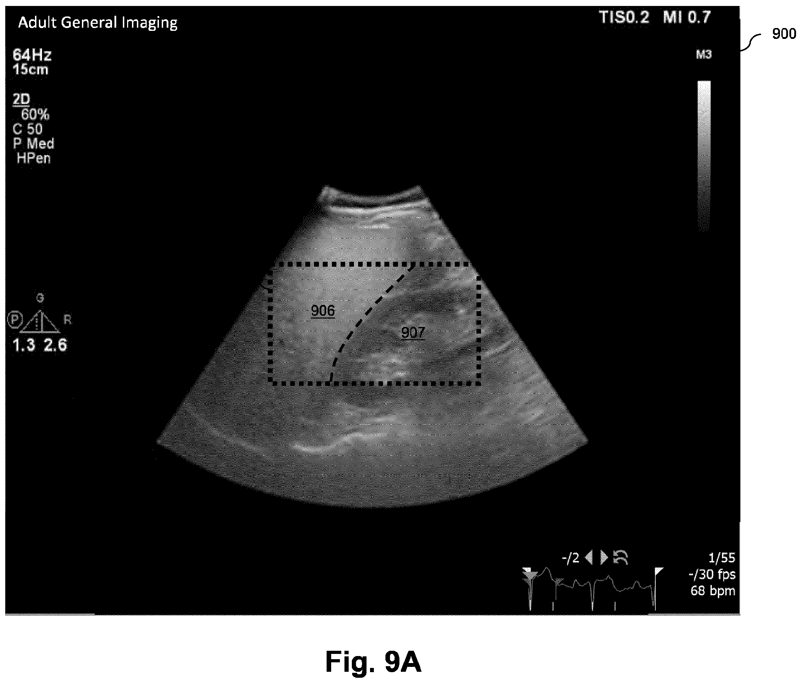

At step 350, the method 300 includes displaying the selected ultrasound image, an indication of the extracted features, and/or the feature score. After each feature is assigned a score at the step 345, the processor circuit 210 may display the results of the score assignment at the step 350. For example, with regard to a kidney-liver contrast feature, multiple images may have been acquired at the step 310 relating to the kidney-liver contrast. At the step 325, one of these ultrasound images may have been selected as the ultrasound image which most strongly correlates to the liver-kidney contrast features. At the step 340, the processor circuit may extract or identify the liver-kidney contrast feature from the selected image. At the step 345, a score may be assigned to the feature. At the step 350, the processor circuit 210 may output to the display (e.g., the display 132) the ultrasound image selected at the step 325 along with an indication that this ultrasound image was selected to show the liver-kidney contrast feature, and the score assigned to the image and/or feature at the step 345. In this way, the user may confirm that the ultrasound image was correctly selected and analyzed. In some aspects, this process of displaying to the user the selected ultrasound image and corresponding feature and feature score may help a non-expert user of the ultrasound image to trust the results of the method 300, thus increasing the likelihood that the method 300 will be more widely adopted. Additional details of the step 350 will be described in greater detail with reference to Figs. 9A-9F hereafter.

At step 355, the method 300 includes determining a staging score or staging value. As will be described with reference to Fig. 8, a machine learning algorithm may be implemented to determine the staging score. In some aspects, the machine learning algorithm which may determine the stage score may the same machine learning algorithm which may be used to extract features from selected ultrasound images and/or the same machine learning algorithm which may be used to assign feature scores to extracted features. The staging value may include a numerical value (e.g., an integer or value within a range) or text (e.g., normal, mild, moderate, or severe).

In some aspects, various mathematical formulae or relationships may be implemented to determine the staging score based on the individual feature scores previously described. In one example, the staging score may be a sum of each of the individual feature scores. For example, the individual feature scores may be added resulting in the staging score. Various ranges of the staging score may determine the stage of NAFLD of the patient. In one example, a staging score of 0-1 may correspond to a normal liver showing no signs of NAFLD. A staging score of 2-3 may indicate mild NAFLD or stage 1 NAFLD. A staging score of 4-5 may indicate moderate or stage 2 NAFLD and a staging score of 6 or more may indicate severe or stage 3 NAFLD. Other relationships may also be established, including the staging score being an average, median, or weighted average of the individual feature scores. In addition, any suitable linear or non-linear relationship may be established between the various features scores and the final staging score. For example, the processor circuit 210 may weight different individual feature scores differently than others depending on the level of correlation between any one feature and the NAFLD stage.

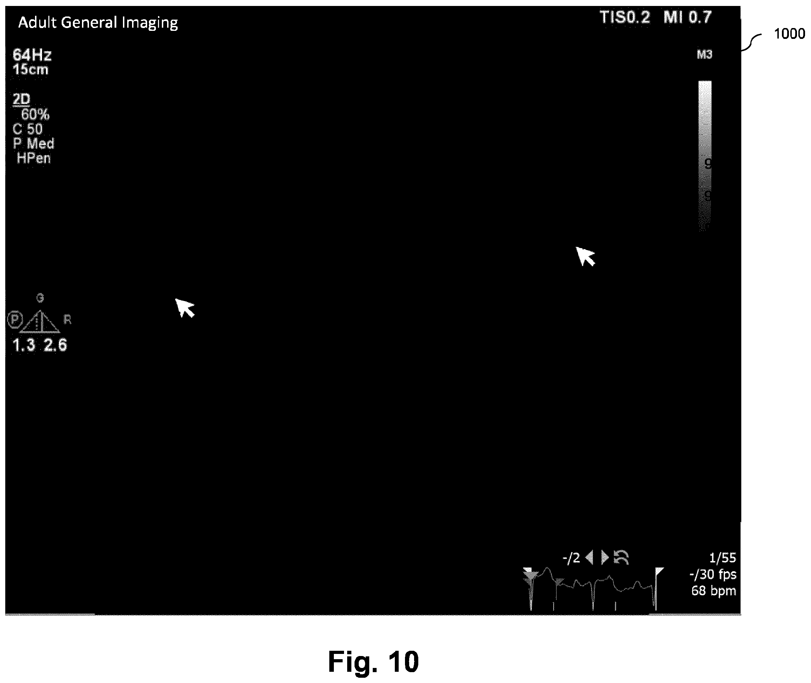

At step 360, the method 300 includes displaying the staging score, one or more ultrasound images, one or more indications of extracted features, and/or one or more feature scores. After the staging score is determined at the step 355, the processor circuit 210 may display the information. Additional aspects of step 360 will be explained in more detail with reference to Figs. 10-11. The processor circuit 210 may be configured to display each of the selected ultrasound images corresponding to each feature as well as the corresponding feature scores of each features. The processor circuit 210 may additionally show the staging score, including the stage (e.g., stage 1, 2, or 3) of NAFLD of the patient. In some aspects, displaying this information may increase the level of trust the user, particular a non-expert user, may have in the results of the method 300.

In some aspects, the processor circuit 210 may additionally generate a report including the results of the method 300 for the user, the patient, the physician, another specialist physician, or any other individual. This report may include a document including the ultrasound images selected, the features, feature scores, the final staging score, and the stage. In some aspects, the report generated may also describe the method of determining the final staging score based on the individual feature scores as well as the method of extracting features and assigning feature scores. In some implementations, an expert physician may review this report to ensure that the method 300 was successfully completed and the staging diagnosis was performed accurately. Additional features and details of the method 300 will be shown and described with reference to the following figures.

Fig. 4 is a diagrammatic view of a graphical user interface 400 displaying user guidance for obtaining ultrasound images for diagnosis, according to aspects of the present disclosure. As described with reference to step 305 of the method 300, user-guidance may include graphical representations of a patient's body with a probe positioned next to the body, text describing the position of the patient and probe, a reference image providing an example of the type of image the user needs to acquire, and a description of expected dynamic movement of the patient anatomy within the expected ultrasound images.

The graphical user interface may include a title 480. The title 480 may indicate to the user of the ultrasound image the type of ultrasound image to be obtained, including the view of the patient anatomy to be obtained, as well as the feature to be extracted from the resulting images. In the example shown in Fig. 4, the graphical user interface 400 may indicate to the user that the user guidance provided within the interface 400 correspond to obtaining an ultrasound image of a first feature (e.g., liver-kidney contrast).

The user guidance within the graphical user interface 400 includes a graphical representation 410. The graphical representation 410 may include a stylized graphic of a patient body 412, and a stylized graphic of an ultrasound transducer probe 420.

The patient body 412 may graphically describe the position of the patient for obtaining ultrasound images of the view, in this case, view A. In the example provided, the user guidance may show the patient in the supine position with the right arm maximally abducted. The probe 420 may be shown adjacent to the patient body 412 at the location 414 relative to the patient anatomy and may illustrate the desired orientation of the probe. In addition, corresponding text 405 may indicate the probe position to the user as well. For example, the text 405 shown in the example of Fig. 4 may indicate that the probe position should be a right intercostal position. In some examples, the text 405 may additionally indicate a plane of view of the ultrasound imaging probe. The plane of view may include a transverse plane, a sagittal plane, or a frontal plane. As shown in Fig. 4, a field of view 425 may be displayed extending from the distal end of the probe 420. This field of view 425 may assist a physician in directing the probe 420 into the patient anatomy.

In some examples, to obtain the view A corresponding to the liver-kidney contrast features and/or any other view or feature described herein, the probe may be positioned such that the hepatic-renal interface is positioned in the middle region of the B-mode ultrasound image. This may help to avoid ultrasound propagation distortion through the inter-organ (e.g., the liver or kidney) boundaries.

In some aspects, additional user guidance may include indicating that the patient should hold their breath during imaging, or any other instructions for the patient, physician, or user. User guidance may include various imaging control parameters, such as intensity, gain, focus, beam steering, time-gain compensation, or any other parameters.

The reference image 455 may additionally be included in the graphical user interface 400. In some aspects, the reference image 455 may be an image of the same region of anatomy from a different patient. The reference image 455 may be an image acquired by an expert user of the ultrasound imaging system. The reference image may be an image showing the view A (e.g., liver-kidney contrast) of the region. The reference image 455 may be a view of a liver-kidney region at any suitable stage of NAFLD. In some aspects, the reference image 455 may be associated with a label 450.

As also shown in Fig. 4, the graphical user interface 400 includes an indication of expected dynamic behavior of the ultrasound images acquired at the desired view. In some aspects, the expected dynamic behavior may include text 460 describing the expected dynamic behavior. For example, the expected dynamic behavior for a particular view may include the diaphragm moving below the liver. **In** some aspects, any other dynamic behavior may be expected. **In** some aspects, the expected dynamic behavior may include a loop of multiple ultrasound images showing movement within the patient anatomy. **In** some aspects, this loop may replace the reference image 455. The reference image 455 may include multiple images obtained in succession at a previous imaging procedure by an expert sonographer. **In** some aspects, the expected dynamic behavior may include an animated image or stylized loop of images showing the expected movement.

As previously mentioned, showing the user of the ultrasound imaging system 100 each of these aspects of the graphical user interface 400 may help the user to feel confident that the imaging procedure is being performed correctly and that the final result (e.g., a NAFLD staging value) is accurate.

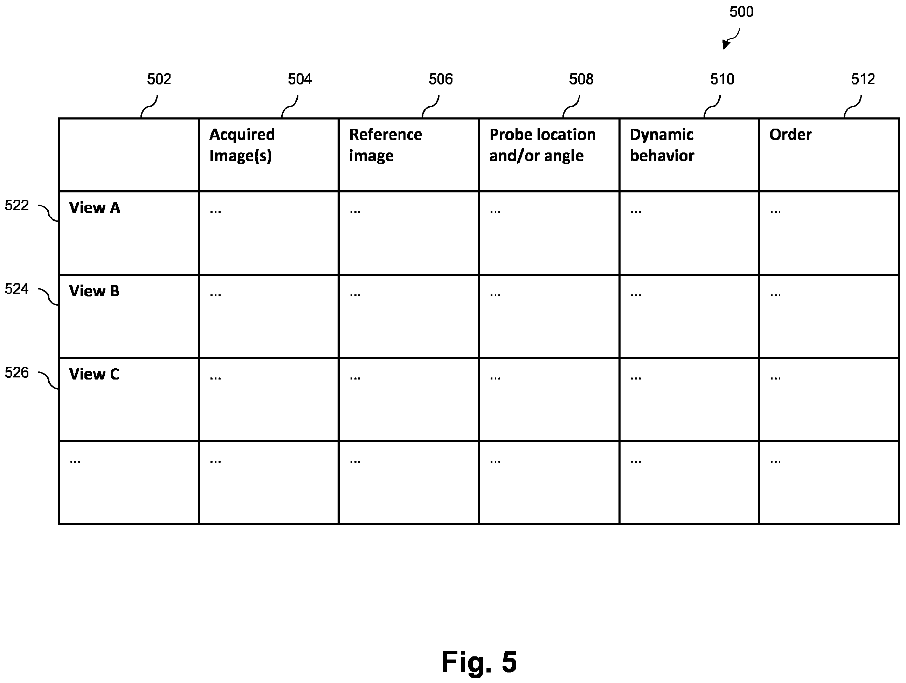

Fig. 5 is a diagrammatic view of a table 500 of user guidance for obtaining ultrasound images of different views for diagnosis, according to aspects of the present disclosure. As shown in Fig. 5, the table 500 may provide a user of the system 100 with a checklist including attributes of the acquired ultrasound images that should be met. It is noted that the table 500 may be organized in any suitable way, including additional rows or columns or having fewer rows or columns as those shown.

A first column 502 of the table 500 includes a list of the views to be obtained. **In** some aspects, the views may include six views corresponding to a liver-kidney contrast, posterior attenuation, portal vein blurring, gallbladder wall visualization, diaphragm definition, and focal fat sparing. It is understood, however, that any number or type of views may be included in the column 502. Each of these views may define a row (e.g., rows 522, 524, and 526, etc.) Each subsequent column may provide the user with an attribute or information related to each of these views.

The column 504 may include an acquired image corresponding to the view. With reference to view A in row 522, the acquired image of column 504 may be an image acquired by the user of the ultrasound imaging system 100 during the present procedure. The acquired image 504 may be compared to the reference image 506 and/or analyzed according to any of the other criteria listed in the table 500.

In some examples, the reference image of column 506 may be the same image as the image 455 of Fig. 4 or may differ depending on the view. The acquired image of column 504 may be compared to the reference image of the column 506 to ensure that the acquired image 504 accurately captures the view of the row 522 and other rows.

The column 508 may include information or directions related to the probe location and/or angle. This information may be of any suitable form including text or visual graphics, images, or symbols. For example, the probe location and/or angle may be substantially similar to the graphical representation 410 shown and described with reference to Fig. 4.

The column 510 may include the expected dynamic behavior of the acquired image or images. The expected dynamic behavior may be shown in any suitable form. For example, the expected dynamic behavior listed in the column 510 may be substantially similar to the expected dynamic behavior 460 described with reference to Fig. 4.

The column 512 may include an order in which the various views are to be acquired. In some aspects, the order may be determined by the user of the ultrasound imaging system 100 prior to the imaging procedure. In some aspects, the order may be provided to the user and may be based on a recommended order. In some aspects, the order of views may not be specified.

The table 500 may be at least a portion of a guidance toolkit provided to the user to help the user to check if the acquired images or sequence is of sufficient quality with clear appearances of the kidney and/or liver, or other structures to ensure that the final staging value determined is accurate.

Fig. 6 is a diagrammatic view of acquired ultrasound images of multiple views and corresponding reference images for comparison, according to aspects of the present disclosure. In some aspects, the multiple ultrasound images may be acquired corresponding to a single view. For example, during the ultrasound imaging procedure, the user of the system 100 may specify the intended view to be captured prior to acquiring ultrasound images. Based on this indication, each ultrasound image acquired after the indication is received may be associated with the particular view.

For example, the user of the ultrasound imaging system 100 may provide a user input indicating that the view A is the intended view to be captured. After this indication is received, a series of ultrasound images 620 may be received. In some aspects, one ultrasound image of the images 620 may be selected as most accurately capturing the view A. For the purposes of the present disclosure, the image which most accurately captures the intended view may be referred to as the key image. To select the key image for the view A, each of the images 620 associated with the view A may be compared with a reference image 630, as shown by the arrow 625. The reference image 630 may be the same image as the reference image 455 shown and described with reference to Fig. 4.

As shown in Fig. 6, the image 622 of the images 620 may be selected as the key image. This image 622 may be selected according to a variety of methods. In one aspect, the image 622 may be selected by the user of the ultrasound imaging system 100. In another aspect, the image 622 may be automatically selected by a processor circuit of the ultrasound imaging system 100. For example, the processor circuit 210 (Fig. 2) may implement various image processing techniques to determine a similarity score between each of the images 620 and the reference image 630. The image 620 with the highest similarity score may be selected as the key image 622. In some aspects, a machine learning network may be used to determine a similarity score for each of the images 620.

In examples in which the key image 622 is automatically selected by the processor circuit 210, the user may be provided with a graphical user interface allowing the user to provide a user input confirming that the key image 622 best represents the view A and corresponds to the reference image 630.

A similar procedure may be performed for each of the views to select a key image for each view. For example, as shown in Fig. 6, ultrasound images 640 may correspond to the view B. Each of these images 640 may be compared to the reference image 650 as shown by the arrow 645. In the example shown, the image 642 may be selected as the key image.

Ultrasound images 660 may correspond to the view C. Each of these images 660 may be compared to the reference image 670 as shown by the arrow 665. In the example shown, the image 642 may be selected as the key image.

It is noted, that the ultrasound image (e.g., the images 620, 640, and 660) may be of any suitable format. For example, the received ultrasound images may be of an ultrasound DICOM image format or raw data in a point of care ultrasound system.

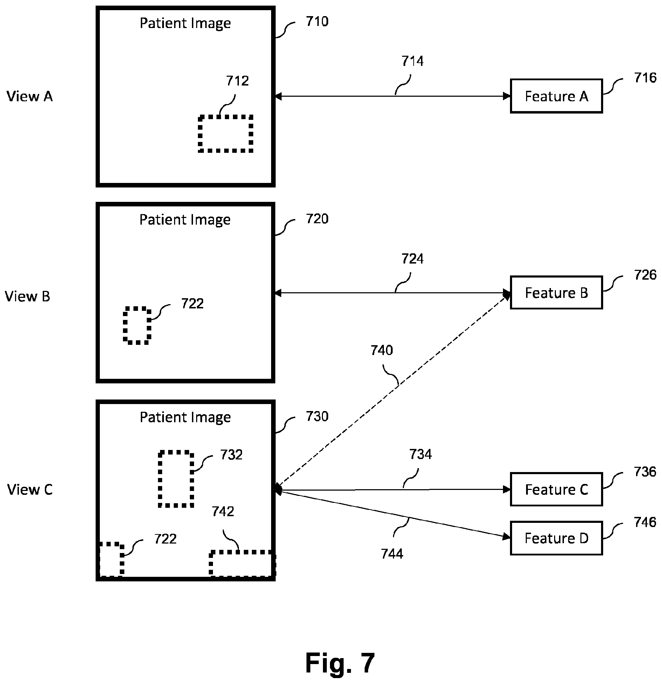

Fig. 7 is a diagrammatic view of acquired ultrasound images and identified features, according to aspects of the present disclosure. As shown in Fig. 7, each of the ultrasound images may correspond to one or more features. In some aspects, each of the views may correspond to one feature. However, an ultrasound image selected as a key image of one particular view may include a feature of that view as well as another feature corresponding to a different view.

For example, a relationship between six different views and six corresponding features may be a one-to-one correspondence. In some aspects, an image corresponding to one view may include multiple features corresponding to multiple views.

Fig. 7 includes an ultrasound image 710. The image 710 may be a key image for view A. In this way, the image 710 may be the same image as the image 622 described with reference to Fig. 6. The image 710 may include the feature 712. For example, the feature 712 may correspond to the liver-kidney contrast. The feature 712 may be extracted from the image 710 as shown by the arrow 714. This feature may be stored as feature A 716 in a memory in communication with the processor circuit 210 and associated with the image 710.

Similarly, Fig. 7 includes an ultrasound image 720. The image 720 may be a key image for view B. In this way, the image 720 may be the same image as the image 642 described with reference to Fig. 6. The image 720 may include the feature 722. For example, the feature 722 may correspond to posterior attenuation. The feature 722 may be extracted from the image 720 as shown by the arrow 724. This feature may be stored as feature B 726 in a memory in communication with the processor circuit 210 and associated with the image 720.

Fig. 7 additionally includes an ultrasound image 730. The image 730 may be a key image for view C. In this way, the image 730 may be the same image as the image 662 described with reference to Fig. 6. The image 730 may include the feature 732. For example, the feature 732 may correspond to portal vein blurring. The feature 732 may be extracted from the image 730 as shown by the arrow 734. This feature may be stored as feature C 736 in a memory in communication with the processor circuit 210 and associated with the image 730.

As shown in Fig. 7, the image 730 may additionally include a portion of the feature 722 as well as a portion of a feature 742. The processor circuit 210 may extract all relevant features from any of the images shown. In the example of view C, shown, the processor circuit 210 may extract the feature 722 as shown by the arrow 740. In some aspects, a relationship between the features B 726 and the image 730 may be established. The processor circuit 210 may assign a score to the feature 722, as will be discussed in more detail hereafter, based on the analysis of the features 722 as shown in the image 720 as well as the image 730.

The feature 742 may be a feature corresponding to a different view, for example, view D. This feature 742 may be associated with a gallbladder wall visualization. The processor circuit 210 may extract the features 742 from the image 730 as shown by the arrow 744. The features may be stored as feature D 746 in a memory in communication with the processor circuit 210 and associated with the image 730. In some aspects, an additional image may correspond to the view D and may also include a depiction of the feature 742.

Fig. 8 is a schematic diagram of a machine learning algorithm 800, according to aspects of the present disclosure. The machine learning algorithm 800 may perform various tasks associated with the ultrasound imaging system 100. For example, the machine learning algorithm 800 may extract features from ultrasound images, as described with reference to Fig. 7, assign scores to each extracted feature, and assign a staging value based on the feature scores. In some aspects, the machine learning algorithm 800 may perform other functions such as selecting key images from a group of ultrasound images associated with a particular view, performing quality checks on received ultrasound images, or any other functions described herein.

The machine learning algorithm 800 may include any suitable type of machine learning algorithm. For example, the machine learning algorithm 800 may include one or more convolutional neural networks (CNNs). Aspects of the machine learning algorithm 800 may be scaled to include any suitable number of CNNs. The machine learning algorithm 800 can be trained for identification of various anatomical landmarks or features within a patient anatomy, including a liver-kidney contrast, posterior attenuation, portal vein blurring, gallbladder wall visualization, diaphragm definition, and focal fat sparing, as well as any other features.

At a step 805 of the algorithm, the machine learning algorithm 800 may receive ultrasound images. The ultrasound images may be key images. In some aspects, the network 800 may receive six key images, each corresponding to at least one view.

At step 810, the preprocessing of the data (e.g., the images received at step 805) may be performed. In some aspects, preprocessing may include adjusting parameters of the input images. For example, various filters may be applied to enhance the visibility of image features. For example, filters applied to the images may increase visibility of the boundary of the liver, vessels within the liver, the diaphragm, focal fat sparing if present, or any other features. Preprocessing may also include contrast enhancement, noise reduction, or any other image alteration. In some aspects, the input images may be resized or otherwise modified prior to extracting image features at the step 815.

At step 815, the machine learning algorithm 800 may extract features, or regions of interest, from each of the images received at step 805. In some aspects, this extraction may include identifying regions within each image which most closely correspond to the view of each received image. As explained with reference to Fig. 7, any of the images of step 805 may be primarily associated with a single view of the patient anatomy. However, any of the images of step 805 may additionally include features corresponding to other views. As shown at the step 820, the primary feature may be extracted from each image of step 805. In other aspects, additional features may be extracted from each image and correlated with each other depending on which view each feature corresponds to.

Step 830 of the machine learning algorithm 800 may correspond to a multitask classification step and may include several layers. These layers may be any suitable number of convolutional layers, fully connected layers, or layers of any other type. In some aspects, each convolutional layer may include a set of filters configured to extract features from the input (e.g., the images of step 805 and/or the features of step 820). The number of layers and filters implemented may vary depending on the embodiments. In some instances, the convolutional layers and the fully connected layers may utilize a leaky rectified non-linear (ReLU) activation function and/or batch normalization. The fully connected layers may be non-linear and may gradually shrink the high-dimensional output to a dimension of the prediction result (e.g., the classification output or layer 855). Thus, the fully connected layers may also be referred to as a classifier. In some embodiments, the fully convolutional layers may additionally be referred to as perception or perceptive layers.

In some aspects, the multitask classification step 830 may include an input layer 840, a shared hidden layer 845, a task-specific hidden layer 850, and an output layer 855. The output layer 855 may include six classifications corresponding to each of the six features input at the input layer 840. In the example shown in Fig. 8, the output layer 855 may determine a score 860 corresponding to feature A (e.g., kidney-liver contrast), a score 862 corresponding to feature B (e.g., posterior attenuation), a score 864 corresponding to feature B (e.g., portal vein blurring), a score 866 corresponding to feature D (e.g., gallbladder wall visualization), a score 868 corresponding to feature E (e.g., diaphragm definition), and a score 870 corresponding to feature F (e.g., focal fat sparing). These scores 860, 862, 864, 866, 868, and 870 may be of a format as those described with reference to step 345 of the method 300 of Fig. 3. However, any suitable format may be used, including any suitable ranges, percentages, ratios, or other values. In some aspect, the machine learning algorithm 800 may additionally determine a confidence level associated with the each feature. This may include the level of confidence with which the network 800 determines that the particular feature is shown within the corresponding image. For the purposes of the present disclosure, the scores 860, 862, 864, 866, 868, and 870 may be referred to as sub-scores. In this way, the sub-scores output from the machine learning algorithm 800 may be a grading of the extent to which a particular medical condition (e.g., NAFLD) is manifesting in the received ultrasound images. In some aspects, as described with reference to Fig. 7, input images of step 805 may include features related to multiple views. As a result, in some aspects, any of the sub-scores 860, 862, 864, 866, 868, and 870 may be based on aspects of not just one input image, but any of the input images which contains information related to the feature of a particular sub-score.

As shown in Fig. 8, a final step of the deep learning algorithm may include determining a staging value 880. The staging value 880 may be the stage of progression of a particular illness or condition being monitored. For example, a stage of 0, 1, 2, or 3 may correspond to an NAFLD condition. The staging value 880 may be of any suitable type and calculated in any suitable way, including those described with reference to step 355 of the method 300 (Fig. 3).

In some aspects, The machine learning algorithm 800 may output a feature vector at the output of the last convolutional layer. The feature vector may indicate objects or features detected from a corresponding input image or other data.

The machine learning algorithm 800 may implement or include any suitable type of learning network. For example, the machine learning algorithm 800 could include a convolutional neural network, a multi-class classification network, an encoder-decoder type network, or any suitable network or means of identifying features within an image.

In an embodiment in which the machine learning algorithm 800 includes an encoder-decoder network, the network may include two paths. One path may be a contracting path, in which a large image, such as an input image of the step 805, may be convolved by several convolutional layers such that the size of the image changes in depth of the network. The image may then be represented in a low dimensional space, or a flattened space. From this flattened space, an additional path may expand the flattened space to the original size of the image. In some embodiments, the encoder-decoder network implemented may also be referred to as a principal component analysis (PCA) method. In some embodiments, the encoder-decoder network may segment the image into patches.

In an additional embodiment of the present disclosure, the machine learning algorithm 800 may include a multi-class classification network. In such an embodiment, the multi-class classification network may include an encoder path. For example, an input image (e.g., one of the images of step 805) may be a high dimensional image. The image may then be processed with the convolutional layers such that the size is reduced. The resulting low dimensional representation of the image may be used to generate a feature vector. The low dimensional representation of the image may additionally be used by the fully connected layers to regress and output one or more classes, such as the features listed in the output layer 855. In some regards, the fully connected layers may process the output of the encoder or convolutional layers. The fully connected layers may additionally be referred to as task layers or regression layers, among other terms.

It is noted that in some aspects, more than six features may be extracted and more than six corresponding images may be received illustrating these features. For example, various types of vein blurring may be included as identified features, including large hepatic vein blurring, main right portal vein blurring, or anterior posterior right portal vein blurring. Additional features may include liver echotexture and overall impression.

In some aspects, the machine learning algorithm 800 my include two separated modules for different tasks. For example, one module may be implemented to extract features from input images. Another module may be implemented to determine the staging value 880. In some aspects, the module for extracting features from input images may be an object detection algorithm, for example Yolov4. In some aspects, the machine learning algorithm 800 may include two separate machine learning algorithms. For example, one machine learning algorithm may be implemented to extract features from input images. Another machine learning algorithm may be implemented to determine sub-scores associated with the extracted features as well as the staging value 880.

Any suitable combination or variations of the machine learning algorithm 800 described is fully contemplated. For example, the machine learning algorithm may include fully convolutional networks or layers or fully connected networks or layers or a combination of the two. In addition, the machine learning algorithm may include a multi-class classification network, an encoder-decoder network, or a combination of the two.

Fig. 9A is a diagrammatic view of a graphical user interface 900 displaying a selected ultrasound image 902 for a particular view 908, a feature 904, and a score 909, according to aspects of the present disclosure.

The ultrasound image 902 included in the graphical user interface 900 may be the key image selected corresponding to the feature of view A. In some aspects, the image 902 may be a different image. In the example shown in Fig. 9A, the feature identified may be the liver-kidney contrast, as shown by the label 908. To identify the feature 904 within the ultrasound image 902, the processor circuit 210 may be configured to overlay a graphical element corresponding to the feature on the ultrasound image 902. In some aspects, the feature 904 may include a region of the liver 906 and a region of the kidney 907.

The graphical user interface 900 may include a label identifying the view A 908 and the corresponding feature (e.g., liver-kidney contrast). An additional label provides the score 909 assigned to the particular feature. The score 909 may be the score 860 described as a result of the output layer 855 of the machine learning algorithm 800. In some aspects, the score 909 may be accompanied by a description of the score. In the graphical user interface 900 provided in Fig. 9A, for example, the score 909 of three may be accompanied by the description, "severely hyperechoic." Varying descriptions may be included corresponding to other values of the score 909.

In some aspects, the graphical user interface 900 may additionally include a region configured to receive a user input, such as a button, text box, or any other form of user input. This user input may allow the user to confirm that the feature 904 correctly identifies the corresponding view A and feature (e.g., liver-kidney contrast). In some aspects, the user may adjust the location of the feature 904 within the image 902, or provide other user inputs.

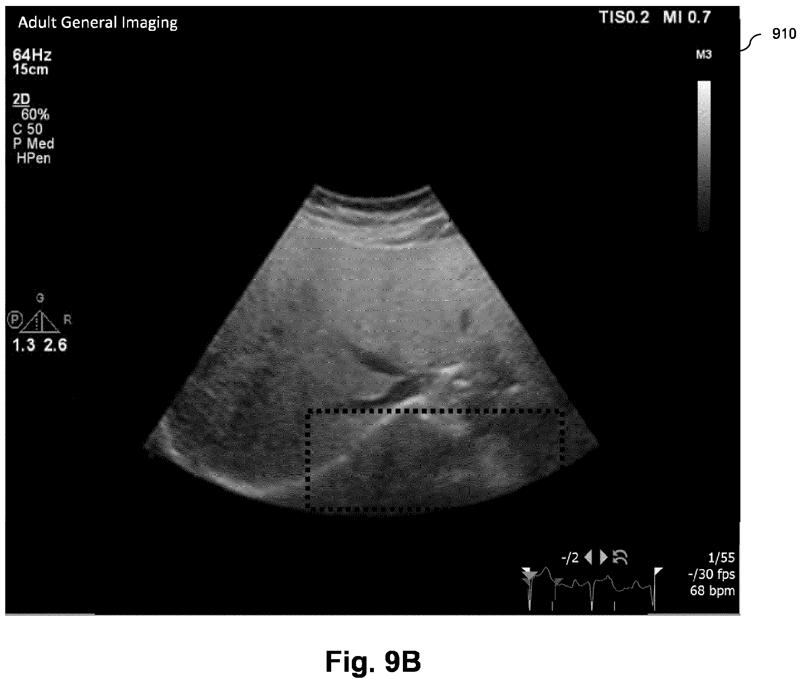

Fig. 9B is a diagrammatic view of a graphical user interface 910 displaying a selected ultrasound image 912 for a particular view 916, a feature 914, and a score 918, according to aspects of the present disclosure.

The ultrasound image 912 included in the graphical user interface 910 may be the key image selected corresponding to the feature of view or may be a different image. In the example shown in Fig. 9B, the feature identified may be posterior attenuation, as shown by the label 916. The processor circuit 210 may overlay a graphical element over the image 912 identifying the feature 914.

The graphical user interface 910 may include a label identifying the view B 916 and the corresponding feature (e.g., posterior attenuation). An additional label provides the score 918 assigned to the particular feature. The score 918 may be the score 862 described as a result of the output layer 855 of the machine learning algorithm 800. In some aspects, the score 918 may be accompanied by a description of the score, such as, "attenuation." Varying descriptions may be included corresponding to other values of the score 918.

Like the graphical user interface 900 of Fig. 9A, the graphical user interface 910 of Fig. 9B may additionally include a region configured to receive a user input confirming that the feature 914 correctly identifies the corresponding view B and feature.

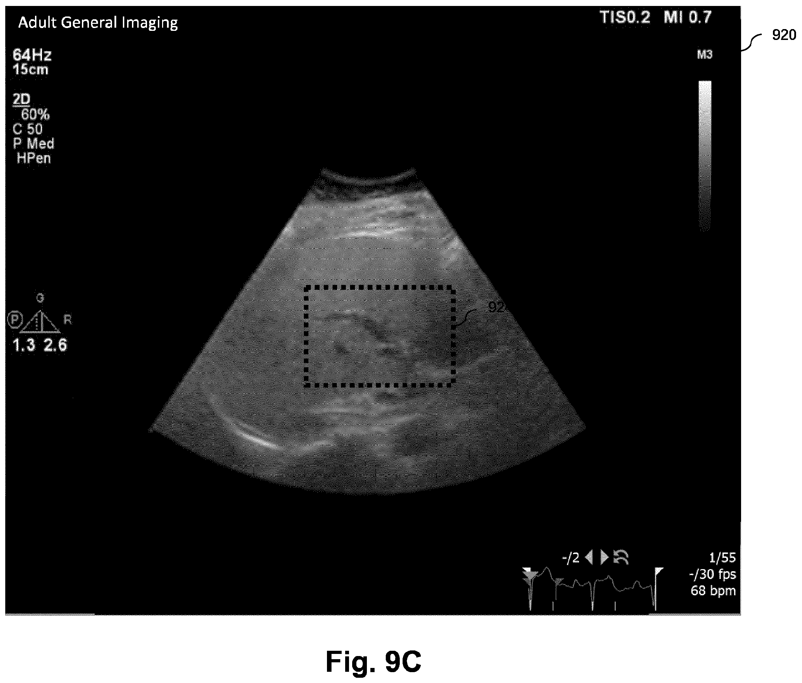

Fig. 9C is a diagrammatic view of a graphical user interface 920 displaying a selected ultrasound image 922 for a particular view 926, a feature 924, and a score 928, according to aspects of the present disclosure.

The ultrasound image 922 included in the graphical user interface 920 may be the key image selected corresponding to the feature of view or may be a different image. In the example shown in Fig. 9C, the feature identified may be portal vein blurring, as shown by the label 926. The processor circuit 210 may overlay a graphical element over the image 922 identifying the feature 924.

The graphical user interface 920 may include a label identifying the view C 926 and the corresponding feature (e.g., portal vein blurring). An additional label provides the score 928 assigned to the particular feature. The score 928 may be the score 864 described as a result of the output layer 855 of the machine learning algorithm 800. In some aspects, the score 928 may be accompanied by a description of the score, such as, "blurring" or varying descriptions corresponding to other values of the score 928. Like the graphical user interfaces previously described, the graphical user interface 920 of Fig. 9C may include a region configured to receive a user input confirming that the feature 924 correctly identifies the corresponding view C and feature.

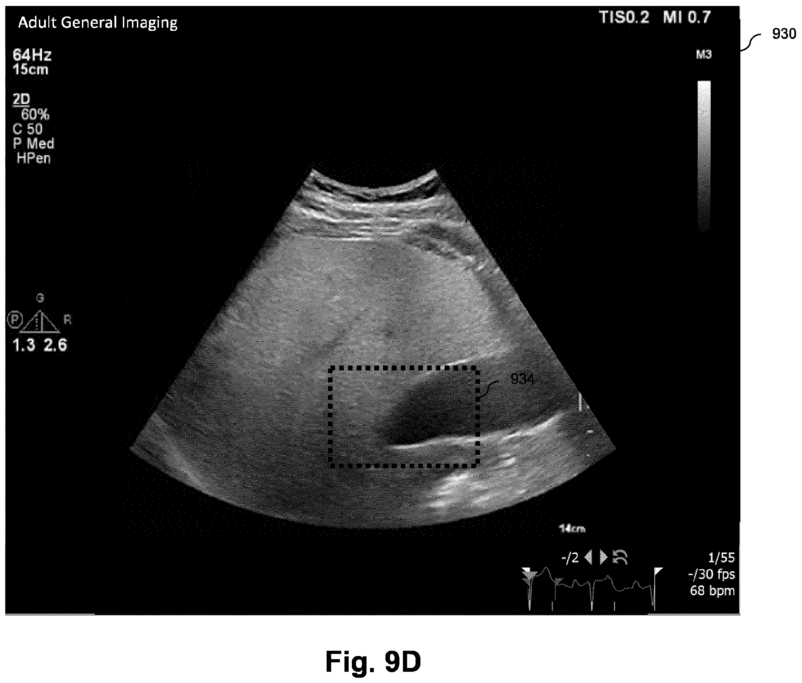

Fig. 9D is a diagrammatic view of a graphical user interface 930 displaying a selected ultrasound image 932 for a particular view 936, a feature 934, and a score 938, according to aspects of the present disclosure.

The ultrasound image 932 included in the graphical user interface 930 may be the key image selected corresponding to the feature of view or may be a different image. **In** the example shown in Fig. 9D, the feature identified may be gallbladder wall visualization, as shown by the label 936. The processor circuit 210 may overlay a graphical element over the image 932 identifying the feature 934.

The graphical user interface 930 may include a label identifying the view D 936 and the corresponding feature (e.g., gallbladder wall visualization). An additional label provides the score 938 assigned to the particular feature. The score 938 may be the score 866 described as a result of the output layer 855 of the machine learning algorithm 800. In some aspects, the score 938 may be accompanied by a description of the score, such as, "poor visualization" or varying descriptions corresponding to other values of the score 938.

Like the graphical user interfaces previously described, the graphical user interface 930 of Fig. 9D may include a region configured to receive a user input confirming that the feature 934 correctly identifies the corresponding view D and feature.

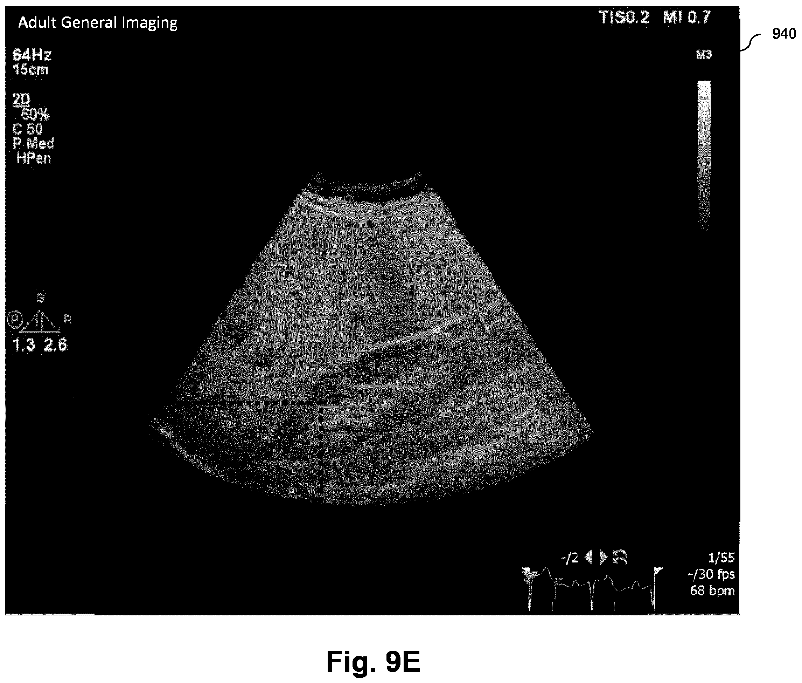

Fig. 9E is a diagrammatic view of a graphical user interface 940 displaying a selected ultrasound image 942 for a particular view 946, a feature 944, and a score 948, according to aspects of the present disclosure.

The ultrasound image 942 included in the graphical user interface 940 may be the key image selected corresponding to the feature of view or may be a different image. In the example shown in Fig. 9E, the feature identified may be diaphragm definition, as shown by the label 946. The processor circuit 210 may overlay a graphical element over the image 942 identifying the feature 944.

The graphical user interface 940 may include a label identifying the view E 946 and the corresponding feature (e.g., gallbladder wall visualization). An additional label provides the score 948 assigned to the particular feature. The score 948 may be the score 868 described as a result of the output layer 855 of the machine learning algorithm 800. In some aspects, the score 948 may be accompanied by a description of the score, such as, "obscured" or varying descriptions corresponding to other values of the score 948.

Like the graphical user interfaces previously described, the graphical user interface 940 of Fig. 9E may include a region configured to receive a user input confirming that the feature 944 correctly identifies the corresponding view E and feature.

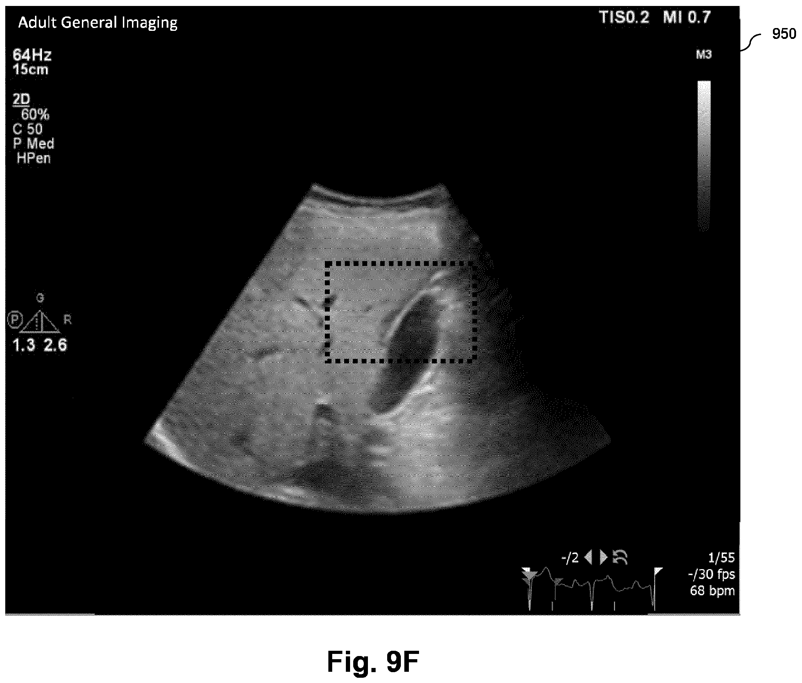

Fig. 9F is a diagrammatic view of a graphical user interface 950 displaying a selected ultrasound image 952 for a particular view 956, a feature 954, and a score 958, according to aspects of the present disclosure.

The ultrasound image 952 included in the graphical user interface 950 may be the key image selected corresponding to the feature of view or may be a different image. In the example shown in Fig. 9F, the feature identified may be focal fat sparing, as shown by the label 956. The processor circuit 210 may overlay a graphical element over the image 952 identifying the feature 954.

The graphical user interface 950 may include a label identifying the view F 956 and the corresponding feature (e.g., focal fat sparing). An additional label provides the score 958 assigned to the particular feature. The score 958 may be the score 870 described as a result of the output layer 855 of the machine learning algorithm 800. In some aspects, the score 958 may be accompanied by a description of the score, such as, "obscured" or varying descriptions corresponding to other values of the score 958. Like the graphical user interfaces previously described, the graphical user interface 950 of Fig. 9F may include a region configured to receive a user input confirming that the feature 954 correctly identifies the corresponding view F and feature.

Figs. 9A-9F may be displayed to a user while the machine learning algorithm 800 is executed. In this way, the user may see the image corresponding to each view, the identified feature within each image, as well as the score associated with each feature. As previously described, the user may input a confirmation of the displayed data and/or results at varying stages. By displaying the images, features, and scores described with reference to Figs. 9A-9F to the user during execution of the deep learning algorithm, the user may be more likely to accept that the final staging value result is accurate and trust the results of the imaging procedure.

Fig. 10 is a diagrammatic view of a graphical user interface 1000 displaying ultrasound images, corresponding scores, and a staging value, according to aspects of the present disclosure.

The graphical user interface 1000 may simultaneously display the six ultrasound images of Figs. 9A-9F. Each of these six ultrasound images may correspond to the six views described previously.

A corresponding list of views, features, and scores is also displayed within the graphical user interface 1000. For example, the label 1031 may identify the view A and may include a description of the view, including, for example, liver-kidney contrast. The score 909, described with reference to Fig. 9A, is also displayed adjacent to the label 1031. The label 1031 and the score 909 may each correspond to the ultrasound image 902.

Similarly, a label 1032 may identify the view B and may include a description of the view, including, for example, posterior attenuation. The score 918, described with reference to Fig. 9B, is also displayed adjacent to the label 1032. The label 1032 and the score 918 may each correspond to the ultrasound image 912. A label 1033 similarly corresponds to the view C and is associated with the score 928 and the ultrasound image 922. A label 1034 corresponds to the view D and is associated with the score 938 and the ultrasound image 932. A label 1035 corresponds to the view E and is associated with the score 948 and the ultrasound image 942. A label 1036 corresponds to the view F and is associated with the score 958 and the ultrasound image 952.

Fig. 10 additionally includes a total score label 1050 and a total score 1052. The total score 1052 may be a combination of the scores 909, 918, 928, 938, 948, and 958 corresponding to the various views A-F. For example, the total score 1052 may be a summation of the scores 909, 918, 928, 938, 948, and 958. In some aspects, the total score 1052 may be a different combination of these scores, such as an average, a median, or any other combination including various relationships or functions.

Fig. 10 also includes an NAFLD stage label 1060 and the staging value 1062. As previously described, the staging value 1062 may be based on the scores 909, 918, 928, 938, 948, and 958. As described with reference to step 355 of the method 300 of Fig. 3, the staging value 1062 may additionally be based on the total score 1052. For example, if the total score 1052 falls within a certain range of values, this range may correspond to one of the staging values. In the example shown, the total score 1052 may be 7 and the NAFLD staging value 1062 may be 3 corresponding to severe progression of NAFLD.

The graphical user interface 1000 may allow the user to select ultrasound images of the images provided as well as views of the views provided. For example, as shown by the indicator 1020, the user may select the ultrasound image 922. In response to this selection, the processor circuit 210 may be configured to modify the graphical user interface 1000 to highlight the selected ultrasound image 922. Similarly, the label 1033 and score 928 may be highlighted because the label 1033 and the score 928 correspond to the ultrasound image 922. As additionally shown by the indicator 1070, the user may alternatively select the label 1033 or the score 928. In response to this selection, the label 1033 and the score 928 may be highlighted as well as the ultrasound image 922 corresponding to the label 1033 and the score of 928.

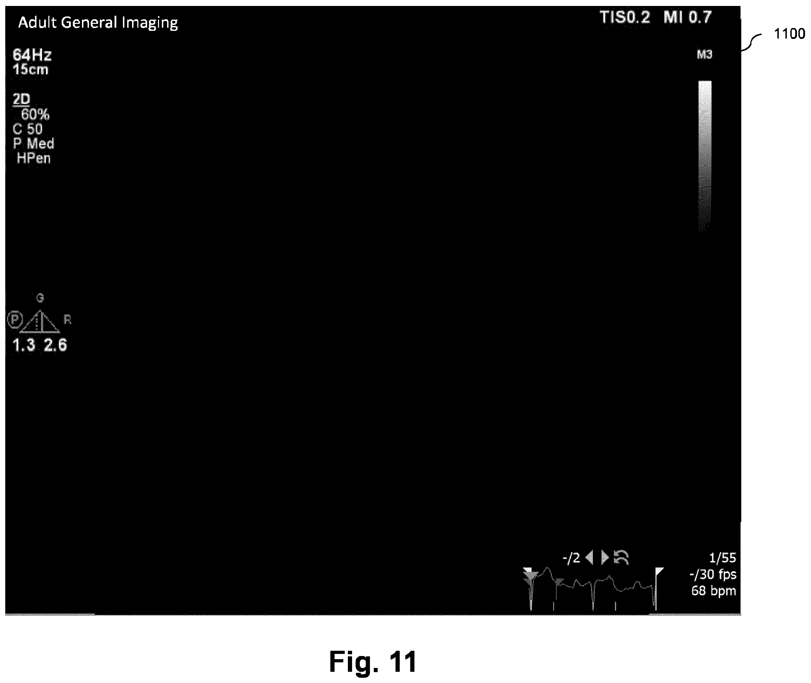

In some aspects, after a user selects an ultrasound image or view within the graphical user interface 1000, the selected ultrasound image may be enlarged as shown and described with reference to Fig. 11.

In some aspects, the processor circuit 210 may additionally be configured to generate a report based on the information displayed in the graphical user interface 1000. The generated report may inform a physician who was not present at the ultrasound imaging procedure of the results. The report may also be viewed by the patient or any other interested parties. The report may include any information, including ultrasound images, overlays, views, features, and scores, as shown and described in Fig. 10 as well as anything else shown and described previously.

Fig. 11 is a diagrammatic view of a graphical user interface 1100 displaying an ultrasound image 922, corresponding scores, and a stage score 1062, according to aspects of the present disclosure.

In some aspects, the graphical user interface 1100 may be displayed to the user in response to the user selecting an ultrasound image or view of the graphical user interface 1000 described previously. For example, the graphical user interface 1100 shown in Fig. 11 may correspond to a view displayed to the user after selecting the ultrasound image 922 corresponding to the view C.

As shown, the graphical user interface 1100 may include an enlarged view of the ultrasound image 922. The ultrasound image 922 may include the feature 924 identified within the image. In some aspects, the ultrasound image 922 may include additional features, such as various metrics or automatically identified features.

The graphical user interface 1100 includes the staging value 1062 as well as a description 1164 of the staging value. in the example shown, because the staging value 1062 is 3, the description 1164 may be "severe."

The graphical user interface 1100 may provide the user with a more detailed view of the image 922. A similar graphical user interface may be displayed corresponding to the other ultrasound images or views of Fig. 10. The graphical user interface 1100 may include any suitable information or detailed data associated with the ultrasound image 922 including any data obtained during the ultrasound imaging procedure. In some aspects, a user of the ultrasound imaging system 100 may view, for example, additional ultrasound images associated with the view C, such as any of the images 660 described with reference to Fig. 6.

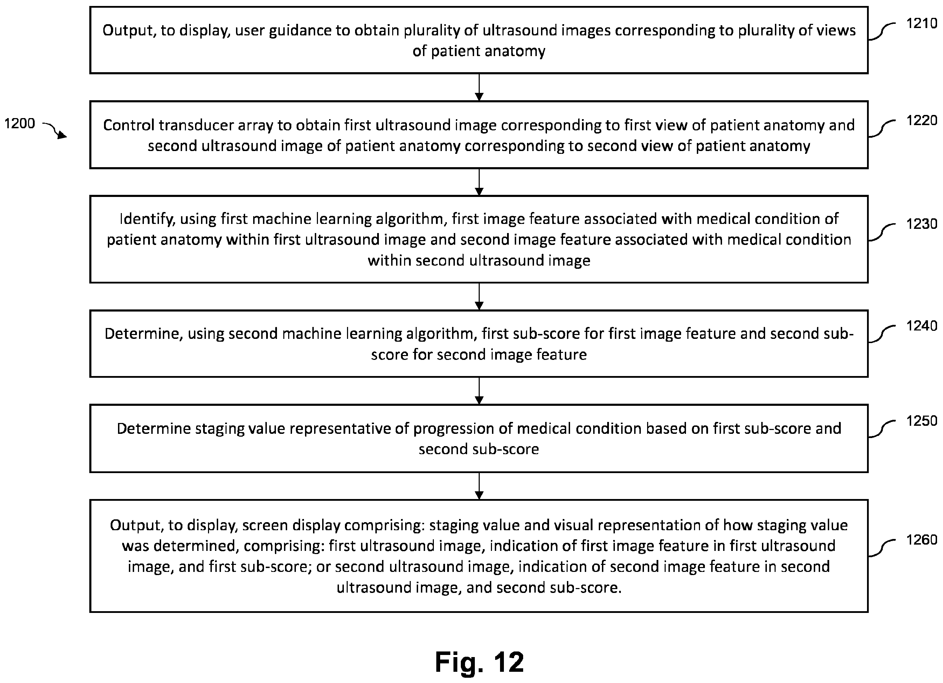

Fig. 12 is a flow diagram of a method of determining a staging value of a medical condition, according to aspects of the present disclosure. As illustrated, the method 1200 includes a number of enumerated steps, but aspects of the method 1200 may include additional steps before, after, or in between the enumerated steps. In some aspects, one or more of the enumerated steps may be omitted, performed in a different order, or performed concurrently. The steps of the method 1200 can be carried out by any suitable component within the system 100 and all steps need not be carried out by the same component. In some aspects, one or more steps of the method 1200 can be performed by, or at the direction of, a processor circuit, including, e.g., the processor 116 (Fig. 1), the processor 134 (Fig. 1), the processor 260 (Fig. 2) or any other suitable component.

At step 1210, the method 1200 includes outputting, to a display, user guidance to obtain a plurality of ultrasound images corresponding to a plurality of views of the patient anatomy. For example, the user guidance may be of any suitable type, including text or visual graphics, including two-dimensional or three-dimensional images, still images, or animated images. User guidance may also include a reference image and/or a description of the expected dynamic behavior of the acquired ultrasound images in real time. Various aspects of user guidance may include any features shown and described with reference to Fig. 4.

At step 1220, the method 1200 includes controlling the transducer array to obtain a first ultrasound image corresponding to a first view of the patient anatomy and a second ultrasound image of the patient anatomy corresponding to a second view of the patient anatomy. Aspects of obtained ultrasound images may be described with reference to step 310 of the method 300 of Fig. 3.

At step 1230, the method 1200 includes identifying, using a first machine learning algorithm, a first image feature associated with a medical condition of the patient anatomy within the first ultrasound image of the plurality of ultrasound images and a second image feature associated with the medical condition within the second ultrasound image of the plurality of ultrasound images. Image features may be extracted from received ultrasound images in any suitable way. For example, a machine learning network may be configured to extract features from input images, as described in more detail with reference to Figs. 7-8.

At step 1240, the method 1200 includes determining, using a second machine learning algorithm, a first sub-score for the first image feature and a second sub-score for the second image feature. The sub-scores assigned to corresponding image features may be of any suitable format or type. Aspects of determining sub-scores associated with identified image features are shown and described with reference to Figs. 9A-9F.

At step 1250, the method 1200 includes determining a staging value representative of the progression of the medical condition based on the first sub-score and the second sub-score. **In** some aspects, the staging value may be a staging value of NAFLD progression within a patient. **In** some aspects, the staging value may be a staging value of another medical condition. Aspects of determining the staging value may be described in greater detail with reference to step 355 of the method 300 of Fig. 3.

At step 1260, the method 1200 includes outputting, to the display, a screen display comprising: the staging value and a visual representation of how the staging value was determined, comprising: the first ultrasound image, the indication of the first image feature in the first ultrasound image, and the first sub-score; or the second ultrasound image, the indication of the second image feature in the second ultrasound image, and the second sub-score. **In** some aspects, the ultrasound images displayed at step 1260 may be displayed sequentially or simultaneously. By displaying this information to a user of the ultrasound imaging system 100 during the imaging procedure, the user may be more confident that the resulting staging value is accurate.

Persons skilled in the art will recognize that the apparatus, systems, and methods described above can be modified in various ways. Accordingly, persons of ordinary skill in the art will appreciate that the aspects encompassed by the present disclosure are not limited to the particular exemplary aspects described above. In that regard, although illustrative aspects have been shown and described, a wide range of modification, change, and substitution is contemplated in the foregoing disclosure. It is understood that such variations may be made to the foregoing without departing from the scope of the present disclosure.

In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements.

## Claims

1. An ultrasound system (100) for determining a staging value representative of a progression of hepatic steatosis of a liver of a patient, the system comprising:
a processor (114, 134) in communication with a display (132) and a transducer array (112) of an ultrasound probe (110), wherein the processor is configured to:
control (1220) the transducer array to obtain a first ultrasound image corresponding to a first view of a patient anatomy and a second ultrasound image of a patient anatomy corresponding to a second, different view of the patient anatomy, wherein the patient anatomy comprises a liver;
identify (1230) a first image feature associated with a medical condition of the patient anatomy within the first ultrasound image and a second, different image feature associated with the medical condition within the second ultrasound image, wherein the medical condition comprises hepatic steatosis;
determine (1240) a first sub-score for the first image feature and a second sub-score for the second image feature;
determine (1250) a staging value representative of a progression of the medical condition based on the first sub-score and the second sub-score; and
output (1260), to the display, a screen display comprising:
the staging value; and
at least one of:
the first ultrasound image, an indication of the first image feature in the first ultrasound image, and the first sub-score; or
the second ultrasound image, an indication of the second image feature in the second ultrasound image, and the second sub-score;.

2. The ultrasound system of claim 1, wherein the processor is further configured to:
output (1210), to the display, user guidance to obtain the first ultrasound image corresponding to the first view of the patient anatomy.

3. The ultrasound system of claim 2, wherein the user guidance comprises a graphical representation of a probe (420) and/or orientation for the ultrasound probe.

4. The ultrasound system of any one of claims 2 to 3, wherein the user guidance comprises a reference image (455) associated with the first view of the patient anatomy.

5. The ultrasound system of any one of claims 2 to 4, wherein the first user guidance comprises a description (460) of a dynamic behavior associated with the first view of the patient anatomy.

6. The ultrasound system of any one of claims 1 to 5, wherein the processor is further configured to determine a quality associated with the first ultrasound image before identifying the first image feature within the first ultrasound image.

7. The ultrasound system of claim 6, wherein the processor is further configured to determine the quality based on a comparison between the first ultrasound image and a reference image associated with the first view of the patient anatomy.

8. The ultrasound system of any one of claims 6 or 7, wherein the processor is further configured to (320):
if the quality satisfies a threshold, identify the first image feature within the first ultrasound image;
if the quality does not satisfy the threshold, control the transducer array to obtain a further ultrasound image corresponding to the first view of the patient anatomy.

9. The ultrasound system of any one of claims 1 to 8, wherein, to determine the first sub-score and the second sub-score, the processor is further configured to implement a first machine learning algorithm.

10. The ultrasound system of claim 9, wherein the first machine learning algorithm comprises a multi-task learning model.

11. The ultrasound system of any one of claims 9 or 10, wherein, to identify the first image feature and the second image feature, the processor is configured to implement a second machine learning algorithm different than the first machine learning algorithm.

12. The ultrasound system of any one of claims 1 to 11, wherein the first sub-score for the first image feature and the second sub-score for the second image feature correspond to ultrasonographic fatty liver indicator.

13. The ultrasound system of any one of claims 1 to 12, wherein the first image feature and the second feature each comprise a different one of: liver-kidney contrast, posterior attenuation, vessel blurring, gallbladder visualization, diaphragmatic attenuation visualization, or focal sparing.

14. A non-transitory computer readable medium storing instructions which, when executed by a processor in communication with a display and a transducer array of an ultrasound probe, cause the processor to execute a method for determining a staging value representative of a progression of hepatic steatosis of a liver of a patient, the method comprising:
controlling (1220) a transducer array of an ultrasound imaging probe to obtain a first ultrasound image corresponding to a first view of a patient anatomy and a second ultrasound image of a patient anatomy corresponding to a second, different view of the patient anatomy, wherein the patient anatomy comprises a liver;
identifying (1230) a first image feature associated with a medical condition of the patient anatomy within the first ultrasound image and a second, different image feature associated with the medical condition within the second ultrasound image, wherein the medical condition comprises hepatic steatosis;
determining (1240) a first sub-score for the first image feature and a second sub-score for the second image feature;
determining (1250) a staging value representative of a progression of the medical condition based on the first sub-score and the second sub-score; and
outputting (1250), to the display, a screen display comprising:
the staging value; and
at least one of:
the first ultrasound image, an indication of the first image feature in the first ultrasound image, and the first sub-score; or
the second ultrasound image, an indication of the second image feature in the second ultrasound image, and the second sub-score.

## Patentansprüche

1. Ultraschallsystem (100) zum Bestimmen eines Staging-Werts, der für eine Progression der hepatischen Steatose einer Leber eines Patienten repräsentativ ist, wobei das System Folgendes umfasst:
einen Prozessor (114, 134) in Kommunikation mit einer Anzeige (132) und einem Wandlerarray (112) einer Ultraschallsonde (110), wobei der Prozessor konfiguriert ist zum:
Steuern (1220) des Wandlerarrays, um ein erstes Ultraschallbild, das einer ersten Ansicht einer Patientenanatomie entspricht, und ein zweites Ultraschallbild einer Patientenanatomie, das einer zweiten, anderen Ansicht der Patientenanatomie entspricht, zu erhalten, wobei die Patientenanatomie eine Leber umfasst;
Identifizieren (1230) eines ersten Bildmerkmals, das mit einem medizinischen Zustand der Patientenanatomie assoziiert ist, innerhalb des ersten Ultraschallbilds, und eines zweiten, anderen Bildmerkmals, das mit dem medizinischen Zustand assoziiert ist, innerhalb des zweiten Ultraschallbilds, wobei der medizinische Zustand hepatische Steatose umfasst;
Bestimmen (1240) eines ersten Teil-Scores für das erste Bildmerkmal und eines zweiten Teil-Scores für das zweite Bildmerkmal;
Bestimmen (1250) eines Staging-Werts, der für eine Progression des medizinischen Zustands repräsentativ ist, basierend auf dem ersten Teil-Score und dem zweiten Teil-Score; und
Ausgeben (1260) einer Bildschirmanzeige, die Folgendes umfasst, an die Anzeige:
den Staging-Wert; und
mindestens eines von:
dem ersten Ultraschallbild, einer Angabe des ersten Bildmerkmals in dem ersten Ultraschallbild und dem ersten Teil-Score; oder
dem zweiten Ultraschallbild, einer Angabe des zweiten Bildmerkmals in dem zweiten Ultraschallbild und dem zweiten Teil-Score.

2. Ultraschallsystem nach Anspruch 1, wobei der Prozessor ferner konfiguriert ist zum:
Ausgeben (1210) einer Benutzerführung an die Anzeige, um das erste Ultraschallbild zu erhalten, das der ersten Ansicht der Patientenanatomie entspricht.

3. Ultraschallsystem nach Anspruch 2, wobei die Benutzerführung eine grafische Darstellung einer Sonde (420) und/oder Orientierung für die Ultraschallsonde umfasst.

4. Ultraschallsystem nach einem der Ansprüche 2 bis 3, wobei die Benutzerführung ein Referenzbild (455) umfasst, das mit der ersten Ansicht der Patientenanatomie assoziiert ist.

5. Ultraschallsystem nach einem der Ansprüche 2 bis 4, wobei die erste Benutzerführung eine Beschreibung (460) eines dynamischen Verhaltens umfasst, das mit der ersten Ansicht der Patientenanatomie assoziiert ist.

6. Ultraschallsystem nach einem der Ansprüche 1 bis 5, wobei der Prozessor ferner dazu konfiguriert ist, eine mit dem ersten Ultraschallbild assoziierte Qualität zu bestimmen, bevor das erste Bildmerkmal innerhalb des ersten Ultraschallbildes identifiziert wird.

7. Ultraschallsystem nach Anspruch 6, wobei der Prozessor ferner dazu konfiguriert ist, die Qualität basierend auf einem Vergleich zwischen dem ersten Ultraschallbild und einem Referenzbild, das mit der ersten Ansicht der Patientenanatomie assoziiert ist, zu bestimmen.

8. Ultraschallsystem nach einem der Ansprüche 6 oder 7, wobei der Prozessor ferner konfiguriert ist zu Folgendem (320):
falls die Qualität eine Schwelle erfüllt, Identifizieren des ersten Bildmerkmals innerhalb des ersten Ultraschallbildes;
falls die Qualität die Schwelle nicht erfüllt, Steuern des Wandlerarrays, um ein weiteres Ultraschallbild zu erhalten, das der ersten Ansicht der Patientenanatomie entspricht.

9. Ultraschallsystem nach einem der Ansprüche 1 bis 8, wobei der Prozessor zum Bestimmen des ersten Teil-Scores und des zweiten Teil-Scores ferner dazu konfiguriert ist, einen ersten Maschinenlernalgorithmus zu implementieren.

10. Ultraschallsystem nach Anspruch 9, wobei der erste Maschinenlernalgorithmus ein Multitasking-Lernmodell umfasst.

11. Ultraschallsystem nach einem der Ansprüche 9 oder 10, wobei der Prozessor zum Identifizieren des ersten Bildmerkmals und des zweiten Bildmerkmals dazu konfiguriert ist, einen zweiten Maschinenlernalgorithmus zu implementieren, der sich von dem ersten Maschinenlernalgorithmus unterscheidet.

12. Ultraschallsystem nach einem der Ansprüche 1 bis 11, wobei der erste Teil-Score für das erste Bildmerkmal und der zweite Teil-Score für das zweite Bildmerkmal einem Ultraschall-Fettleberindikator entsprechen.

13. Ultraschallsystem nach einem der Ansprüche 1 bis 12, wobei das erste Bildmerkmal und das zweite Merkmal jeweils ein anderes von Folgendem umfassen: Leber-Nieren-Kontrast, posteriore Abschwächung, Gefäßunschärfe, Gallenblasenvisualisierung, Visualisierung der Zwerchfellabschwächung, oder fokale Minderverfettung (focal sparing).

14. Nichtflüchtiges computerlesbares Medium, das Anweisungen speichert, die bei Ausführung durch einen Prozessor in Kommunikation mit einer Anzeige und einem Wandlerarray einer Ultraschallsonde bewirken, dass der Prozessor ein Verfahren zum Bestimmen eines Staging-Werts ausführt, der für eine Progression der hepatischen Steatose einer Leber eines Patienten repräsentativ ist, wobei das Verfahren Folgendes umfasst:
Steuern (1220) eines Wandlerarrays einer Ultraschallbildgebungssonde, um ein erstes Ultraschallbild, das einer ersten Ansicht einer Patientenanatomie entspricht, und ein zweites Ultraschallbild einer Patientenanatomie, das einer zweiten, anderen Ansicht der Patientenanatomie entspricht, zu erhalten, wobei die Patientenanatomie eine Leber umfasst;
Identifizieren (1230) eines ersten Bildmerkmals, das mit einem medizinischen Zustand der Patientenanatomie assoziiert ist, innerhalb des ersten Ultraschallbilds, und eines zweiten, anderen Bildmerkmals, das mit dem medizinischen Zustand assoziiert ist, innerhalb des zweiten Ultraschallbilds, wobei der medizinische Zustand hepatische Steatose umfasst;
Bestimmen (1240) eines ersten Teil-Scores für das erste Bildmerkmal und eines zweiten Teil-Scores für das zweite Bildmerkmal;
Bestimmen (1250) eines Staging-Werts, der für eine Progression des medizinischen Zustands repräsentativ ist, basierend auf dem ersten Teil-Score und dem zweiten Teil-Score; und
Ausgeben (1250) einer Bildschirmanzeige an die Anzeige, die Folgendes umfasst:
den Staging-Wert; und
mindestens eines von:
dem ersten Ultraschallbild, einer Angabe des ersten Bildmerkmals in dem ersten Ultraschallbild und dem ersten Teil-Score; oder
dem zweiten Ultraschallbild, einer Angabe des zweiten Bildmerkmals in dem zweiten Ultraschallbild und dem zweiten Teil-Score.

## Revendications

1. Système à ultrasons (100) pour déterminer une valeur de stadification représentative d'une progression d'une stéatose hépatique d'un foie d'un patient, le système comprenant :
un processeur (114, 134) en communication avec un écran (132) et un réseau de transducteurs (112) d'une sonde à ultrasons (110), le processeur étant configuré pour :
commander (1220) le réseau de transducteurs pour obtenir une première image ultrasonore correspondant à une première vue de l'anatomie d'un patient et une seconde image ultrasonore de l'anatomie d'un patient correspondant à une seconde vue différente de l'anatomie du patient, l'anatomie du patient comprenant un foie ;
identifier (1230) une première caractéristique d'image associée à un état médical de l'anatomie du patient dans la première image ultrasonore et une seconde caractéristique d'image différente associée à l'état médical dans la seconde image ultrasonore, l'état médical comprenant une stéatose hépatique ;
déterminer (1240) un premier sous-score pour la première caractéristique d'image et un second sous-score pour la seconde caractéristique d'image ;
déterminer (1250) une valeur de stadification représentative d'une progression de l'état médical sur la base du premier sous-score et du second sous-score ; et
envoyer (1260), à l'écran, un affichage d'écran comprenant :
la valeur de stadification ; et
au moins l'un parmi :
la première image ultrasonore, une indication de la première caractéristique d'image dans la première image ultrasonore et le premier sous-score ; ou
la seconde image ultrasonore, une indication de la seconde caractéristique d'image dans la seconde image ultrasonore et le second sous-score.

2. Système à ultrasons selon la revendication 1, dans lequel le processeur est en outre configuré pour :
envoyer (1210), à l'écran, un guidage utilisateur pour obtenir la première image ultrasonore correspondant à la première vue de l'anatomie du patient.

3. Système à ultrasons selon la revendication 2, dans lequel le guidage utilisateur comprend une représentation graphique d'une sonde (420) et/ou d'une orientation pour la sonde à ultrasons.

4. Système à ultrasons selon l'une quelconque des revendications 2 à 3, dans lequel le guidage utilisateur comprend une image de référence (455) associée à la première vue de l'anatomie du patient.

5. Système à ultrasons selon l'une quelconque des revendications 2 à 4, dans lequel le premier guidage utilisateur comprend une description (460) d'un comportement dynamique associé à la première vue de l'anatomie du patient.

6. Système ultrasonore selon l'une quelconque des revendications 1 à 5, dans lequel le processeur est en outre configuré pour déterminer une qualité associée à la première image ultrasonore avant d'identifier la première caractéristique d'image à l'intérieur de la première image ultrasonore.

7. Système ultrasonore selon la revendication 6, dans lequel le processeur est en outre configuré pour déterminer la qualité sur la base d'une comparaison entre la première image ultrasonore et une image de référence associée à la première vue de l'anatomie du patient.

8. Système à ultrasons selon l'une quelconque des revendications 6 ou 7, dans lequel le processeur est en outre configuré pour (320) :
si la qualité satisfait à un seuil, identifier la première caractéristique d'image dans la première image ultrasonore ;
si la qualité ne satisfait pas au seuil, commander le réseau de transducteurs pour obtenir une autre image ultrasonore correspondant à la première vue de l'anatomie du patient.

9. Système à ultrasons selon l'une quelconque des revendications 1 à 8, dans lequel, pour déterminer le premier sous-score et le second sous-score, le processeur est en outre configuré pour mettre en œuvre un premier algorithme d'apprentissage automatique.

10. Système à ultrasons selon la revendication 9, dans lequel le premier algorithme d'apprentissage automatique comprend un modèle d'apprentissage multitâche.

11. Système à ultrasons selon l'une quelconque des revendications 9 ou 10, dans lequel, pour identifier la première caractéristique d'image et la seconde caractéristique d'image, le processeur est configuré pour mettre en œuvre un second algorithme d'apprentissage machine différent du premier algorithme d'apprentissage machine.

12. Système à ultrasons selon l'une quelconque des revendications 1 à 11, dans lequel le premier sous-score pour la première caractéristique d'image et le second sous-score pour la seconde caractéristique d'image correspondent à un indicateur échographique de foie gras.

13. Système à ultrasons selon l'une quelconque des revendications 1 à 12, dans lequel la première caractéristique d'image et la deuxième caractéristique comprennent chacune une caractéristique différente parmi : le contraste foie-rein, l'atténuation postérieure, le flou vasculaire, la visualisation de la vésicule biliaire, la visualisation de l'atténuation diaphragmatique, ou la réserve focale.

14. Support non transitoire lisible par ordinateur stockant des instructions qui, lorsqu'elles sont exécutées par un processeur en communication avec un écran et un réseau de transducteurs d'une sonde à ultrasons, amènent le processeur à exécuter un procédé pour déterminer une valeur stadification représentative d'une progression d'une stéatose hépatique d'un foie d'un patient, le procédé comprenant :
la commande (1220) d'un réseau de transducteurs d'une sonde d'imagerie ultrasonore pour obtenir une première image ultrasonore correspondant à une première vue de l'anatomie d'un patient et une seconde image ultrasonore de l'anatomie d'un patient correspondant à une seconde vue différente de l'anatomie du patient, l'anatomie du patient comprenant un foie ;
l'identification (1230) d'une première caractéristique d'image associée à un état médical de l'anatomie du patient dans la première image ultrasonore et d'une seconde caractéristique d'image différente associée à l'état médical dans la seconde image ultrasonore, l'état médical comprenant une stéatose hépatique ;
la détermination (1240) d'un premier sous-score pour la première caractéristique d'image et d'un second sous-score pour la seconde caractéristique d'image ;
la détermination (1250) d'une valeur de stadification représentative d'une progression de l'état médical sur la base du premier sous-score et du second sous-score ; et
l'envoi (1250), à l'écran, d'un affichage d'écran comprenant :
la valeur de stadification ; et
au moins l'un parmi :
la première image ultrasonore, une indication de la première caractéristique d'image dans la première image ultrasonore et le premier sous-score ; ou
la seconde image ultrasonore, une indication de la seconde caractéristique d'image dans la seconde image ultrasonore et le second sous-score.
